# EUROPÄISCHE PATENTSCHRIFT

(11) **EP 2 910 270 B1**
(45) Veröffentlichungstag und Bekanntmachung des Hinweises auf die Patenterteilung: **02.08.2017**
(21) Anmeldenummer: 15155993.7
(22) Anmeldetag: 20.02.2015
(51) Int. Cl.: A61M 11/02, A61C 17/028, A61M 3/02, F04B 7/04, F04B 9/127

(54) **Vakuummotor zum Betreiben eines Lavage-Systems**
Vacuum motor for operation of a lavage system
Moteur à vide destiné au fonctionnement d'un système de lavage

(30) Priorität: 24.02.2014 DE 102014203246
(43) Veröffentlichungstag der Anmeldung: 26.08.2015
(73) Patentinhaber: Heraeus Medical GmbH, 61273 Wehrheim (DE)
(72) Erfinder: Vogt, Sebastian, 99092 Erfurt (DE)
(74) Vertreter: Schultheiss & Sterzel Patentanwälte PartG mbB

(56) Entgegenhaltungen:
- EP-A1- 2 873 856
- DE-A1- 3 724 110
- DE-B3-102010 046 057
- US-A- 5 542 918
- US-A- 5 554 011

## Beschreibung

Die Erfindung betrifft einen Vakuummotor aufweisend einen Arbeitskolben und einen Innenraum, in dem der Arbeitskolben linear beweglich angeordnet ist. Die Erfindung betrifft auch ein Lavage-System mit einem solchen Vakuummotor.

Die Erfindung betrifft ferner die Verwendung eines solchen Vakuummotors und ein Verfahren zum Erzeugen einer periodischen Bewegung mit einem Vakuum oder einem Unterdruck sowie ein Verfahren zum Erzeugen eines Sprühstoßes mit einem solchen Verfahren.

Gegenstand der Erfindung ist somit ein vereinfachter Vakuummotor, der im Wesentlichen aus kostengünstigen Kunststoffen gefertigt werden kann und zum Antrieb von nur einmalig, kurzzeitig betriebenen Vorrichtungen, insbesondere von aus Kunststoffen gefertigten, einmalig einsetzbaren medizinischen Spülvorrichtungen bestimmt ist. Es wird ferner eine durch den Vakuummotor angetriebene Pumpe für Flüssigkeiten beschrieben. Weiterhin wird die Verwendung des Vakuummotors zum Antrieb einer Pumpe zum Austragen von Spülflüssigkeit einer medizinischen Spülvorrichtung (das heißt eines Lavage-Systems) vorgeschlagen, die zum nur einmaligen Gebrauch bestimmt ist.

In der Chirurgie werden medizinische Spülsysteme in breitem Umfang eingesetzt, um Gewebeareale zu reinigen. Diese Spülsysteme werden als Lavage-Systeme bezeichnet. Mit den Lavage-Systemen werden mit den Spülflüssigkeiten Sprühstrahlen erzeugt, die auf die zu reinigenden Gewebeareale auftreffen und eine mechanische Reinigungswirkung auf diese Gewebeareale ausüben. Insbesondere bei der Implantation von Gelenkendoprothesen und bei septischen Revisionen sind Lavage-Systeme von wesentlicher Bedeutung (R. M. Sherman et al.: The role of lavage in preventing hemodynamic and blood-gas changes during cemented arthroplasty. J. Bone Joint. Surg. 1983; 65-A: 500-506; S. J. Breusch et al.: Zementierte Hüftendoprothetik: Verminderung des Fettembolierisikos in der zementierten Hüftendoprothetik mittels gepulster Druckspülung. Orthopädie 2000; 29: 578-586; S. J. Breusch et al.: Lavage technique in THA: Jet-lavage Produces Better Cement Penetration Than Syringe-Lavage in the Proximal Femur. J. Arthroplasty. 200; 15(7): 921-927; R. J. Byrick et al.: High-volume, high pressure pulsatile lavage during cemented arthroplasty. J. Bone Joint Surg. 1989; 81-A: 1331-1336; J. Christie et al.: Medullary lavage reduces embolic phenomena and cardiopulmonary changes during cemented hemiarthorplasty. J. Bone Joint Surg. 1995; 77-B: 456-459).

Gepulste Lavage-Systeme sind seit langem bekannt, beispielsweise aus der US 4,583,531 A, der US 4,278,078 A und der US 5,542,918 A. Die gegenwärtig im Markt befindlichen Lavage-Systeme werden durch Elektromotoren (zum Beispiel InterPulse^{®} Jet Lavage der Stryker GmbH & Co. KG) oder durch Druckluft (zum Beispiel PALAVAGE^{®} der Heraeus Medical GmbH) angetrieben. Bewährt haben sich auch handgehaltene, elektrisch angetriebene Lavage-Systeme. Es muss jedoch immer ein großer Batterieblock oder Akkumulatorenblock mitgeführt werden, der naturgemäß nur eine begrenzte Ladungskapazität hat. Batterie- und Akkumulatorenblöcke sind im Hinblick auf ihre Umweltfreundlichkeit kritisch zu sehen. Druckgasgetriebene Lavage-Systeme haben den Vorteil, dass Druckluft im Operationssaal meist unlimitiert zur Verfügung steht und damit beliebig lange Spülflüssigkeit versprüht werden kann, ohne dass die Energiezufuhr begrenzt ist.

Bei der Verwendung von Druckluft-getriebenen Lavage-Systemen muss jedoch eine Doppelschlauchsystem verwendet werden, bei dem in einem Schlauch die unsterile Druckluft zugeführt wird und einen zweiten Schlauch mit dem die nach dem Antrieb des Druckluftmotors zumindest teilentspannte unsterile Luft abgeführt wird. Bei den mit Druckluft oder einem anderen komprimierten Gas getriebenen Systemen wird jedoch üblicherweise ein Druckgasmotor zum Antrieb eingesetzt. Bei den meisten Druckgasmotoren für Lavage-Systeme handelt es sich um Lamellen-Druckgasmotoren. Der Druckgasmotor erzeugt eine Drehbewegung, die dann in eine oszillierende, lineare Bewegung umgesetzt wird. Die oszillierende lineare Bewegung wird genutzt, um jeweils kleinen Volumina des Spülmediums einen Impuls zu verleihen. Dabei wird üblicherweise mindestens eine Membran zwischen dem Antrieb und dem Zulauf der Spülflüssigkeit angeordnet, um die Impulse auf die Spülflüssigkeit übertragen zu können. Es entstehen dadurch Sprühstöße mit hohen Pulszahlen von 2000 bis 3000 Impulsen pro Minute. Das bedeutet, dass der Druckgasmotor sehr präzise gefertigt sein muss, um entsprechend hohe Drehzahlen zu tolerieren. Weiterhin muss eine entsprechend stabile Lagerung vorhanden sein. Aus diesen Gründen ist der Druckgasmotor bei üblichen Druckgasgetriebenen Lavage-Systemen das kostenintensivste Bauteil. Es wird deshalb im Allgemeinen der Druckgasmotor in einem Handgriff aus Metall oder anderen langzeitstabilen Materialien angeordnet, so dass dieses Bauteil mehrfach nach entsprechender Aufbereitung und Sterilisation genutzt werden kann. Druckgasmotoren nutzen den Druckunterschied zwischen dem zum Antrieb eingesetzten komprimierten Gas und der Druck der umgebenden Atmosphäre.

Ein Druckgasmotor ist aus der WO 2012/038003 A1 bekannt. Der dort beschriebene Druckgasmotor hat einen zweiteiligen Kolben mit einem Zwischenraum und einen Durchgang durch einen der Kolben. Dadurch ist der Motor besonders einfach und kostengünstig im Aufbau.

Die US 5 554 011 A offenbart eine Pumpe für ein medizinisches Fluid, die mit einem Vakuum angetrieben wird, das auf eine Membran im Inneren der Pumpe einwirkt.

Bei einer Vielzahl von Operationen ist ein Absaugen von Wundsekret und Blut notwendig. Für das Absaugen dieser Flüssigkeiten werden Absaugvorrichtungen eingesetzt, die mit Unterdruck betrieben werden. Für den Betrieb dieser Vorrichtungen gibt es dazu in den meisten Operationssälen (OPs) stationäre Vakuumanlagen, die üblicherweise einen Unterdruck von 0,8 bar bis 0,9 bar haben. Daneben werden auch mobile Vakuumbeziehungsweise Unterdruck-erzeugende Absauganalgen in breitem Umfang eingesetzt.

Es besteht immer der Wunsch nach einem kostengünstiger aufzubauenden Motor. Zudem besteht auch ein Bedürfnis danach, einen Motor bereitzustellen, der mit einer höheren Frequenz und/oder einer größeren Kraft betrieben werden kann.

Vakuummotoren sind aber nicht nur für Lavage-Systeme geeignet, sondern können auch überall dort eingesetzt werden, wo ein Vakuum oder eine Unterdruckquelle zur Verfügung steht und ein kostengünstiger Antrieb vorteilhaft ist. Solche Voraussetzungen sind beispielsweise bei Rüttelanlagen gegeben, bei denen ein Schüttgut oder Pulver transportiert, abgefüllt beziehungsweise portioniert werden muss. Ebenso können solche Druckgasmotoren als Pumpen zur Bereitstellung von Schmiermitteln vorteilhaft eingesetzt werden.

Die Aufgabe der Erfindung besteht also darin, die Nachteile des Stands der Technik zu überwinden. Insbesondere soll ein kostengünstiger und zuverlässiger Vakuummotor gefunden werden, der für die genannten Zwecke einsetzbar ist. Eine Aufgabe der Erfindung ist dabei darin zu sehen, einen maximal vereinfachten Kolben-Vakuummotor zu entwickeln, der eine periodische, lineare Kolbenbewegung erzeugen kann. Der Vakuummotor soll mit dem Vakuum der, in Operationssälen zur Absaugung von Wundsekret und Blut üblichen, stationären Vakuumanlagen und auch mit mobilen Absauganlagen betrieben werden können. Es soll ferner ein Verfahren zur Erzeugung einer linearen, periodischen Bewegung entwickelt werden, wobei der zu entwickelnde Vakuummotor verwendet werden soll oder zumindest verwendbar sein soll. Dabei ist es wichtig, dass der Vakuummotor ohne komplexe, kostenintensive Ventilsysteme auskommt und so vereinfacht wird, dass die Bauteile des Vakuummotors kostengünstig durch Kunststoffspritzgießen hergestellt werden können. Der Vakuummotor soll für den Antrieb einer medizinischen Spülvorrichtung verwendbar sein. Es müssen Ventilsysteme vermieden werden, die ein großes Volumen einnehmen und die separat vom Vakuummotor positioniert werden müssen. In dem Vakuummotor sollen daher die erforderlichen Ventilfunktionen möglichst platzsparend integriert sein, um eine Verwendung des Vakuummotors als Antrieb in Handstücken von Lavage-Systemen zu ermöglichen. Die zeitliche Steuerung der Ventilfunktionen sollte idealerweise so sein, dass bei jedem Punkt der Kolbenbewegung ein "toter Punkt" vermieden wird. Weiterhin soll mit dem Vakuummotor eine vereinfachte Pumpe entwickelt werden, die in handgehaltenen Lavage-Systemen untergebracht werden kann. Eine mit dem Vakuummotor angetriebene Pumpe soll so vereinfacht sein, das diese derart kostengünstig hergestellt werden kann, dass eine Verwendung in Lavage-Systemen möglich ist, die zur nur einmaligen Verwendung bestimmt sind.

Die Aufgaben der Erfindung werden gelöst durch einen Vakuummotor aufweisend einen Arbeitskolben, einen Innenraum, in dem der Arbeitskolben linear beweglich angeordnet ist, ein Rückstellelement, das zumindest zeitweise eine in Richtung einer Vorderseite des Innenraums wirkende Kraft auf den Arbeitskolben ausübt, eine Gaseinlassöffnung zum Einspeisen von Umgebungsluft oder eines unter Druck stehenden Gases in den Innenraum und eine Gasauslassöffnung zum Abführen des Gases aus dem Innenraum, wobei die Gasauslassöffnung an eine Unterdruckquelle anschließbar ist, wobei zwischen dem Arbeitskolben und einer Rückseite des Innenraums ein Steuerkolben linear beweglich im Innenraum angeordnet ist, wobei der Steuerkolben in einer ersten Position die Gasauslassöffnung nicht abdeckt und die Gaseinlassöffnung abdeckt und in einer zweiten Position die Gaseinlassöffnung nicht abdeckt und die Gasauslassöffnung abdeckt, der Steuerkolben gegen den Arbeitskolben beweglich gelagert ist und an dem Arbeitskolben und/oder dem Steuerkolben ein Mitnehmerelement und/oder ein Abstandhalter angeordnet ist, wobei das Mitnehmerelement bei einer Bewegung des Arbeitskolbens zur Vorderseite des Innenraums hin den Steuerkolben in die erste Position überführt und wobei das Mitnehmerelement oder der Abstandhalter bei einer Bewegung des Arbeitskolbens zur Rückseite des Innenraums hin den Steuerkolben in die zweite Position überführt, wobei der Steuerkolben in einer dritten Position zwischen der ersten Position und der zweiten Position sowohl die Gaseinlassöffnung als auch die Gasauslassöffnung abdeckt.

Unter einem Vakuummotor ist im Rahmen der vorliegenden Erfindung ein Motor zu verstehen, der mit Hilfe eines Druckgefälles zwischen einem Gas mit Unterdruck und einem Gas mit einem höheren Druck Arbeit leisten kann. Dabei wird als Arbeitsmedium ein Gas bei Luftdruck, Normaldruck oder höher verwendet, bevorzugt Luft aus der Umgebung des Vakuummotors verwendet, und das Gas oder die Luft durch den Vakuummotor mit Hilfe des Vakuums beziehungsweise des Unterdrucks geleitet. Durch die Strömung des Gases, bevorzugt der Luft, durch den Vakuummotor wird der Arbeitskolben in eine periodische Bewegung versetzt. Ein Unterdruck ist bereits dann gegeben, wenn der Umgebungsdruck höher ist als der Unterdruck. Ein Differenzdruck von 100 hPa kann im Extremfall ausreichen. Erfindungsgemäß bevorzugt weist die Quelle für den Unterdruck jedoch einen Differenzdruck von 500 hPa oder mehr beziehungsweise einen absoluten Druck von 500 hPa oder weniger auf.

Bevorzugt bewirkt das Rückstellelement bei jeder Stellung des Arbeitskolbens eine Kraft in Richtung der Vorderseite des Innenraums auf den Arbeitskolben.

Bei einem entsprechend vertikal aufgestellten Vakuummotor (Vorderseite des Vakuummotors unten Richtung Erdboden) kann sogar auf ein körperliches Rückstellelement verzichtet werden, da die Rückstellung des Arbeitskolbens dann durch die Schwerkraft erfolgen kann. Es kann also erfindungsgemäß vorgesehen sein, dass das Rückstellelement gebildet wird durch die vertikale Aufstellung des Vakuummotors, durch die der Arbeitskolben mit Hilfe der Schwerkraft zur Vorderseite beschleunigt wird.

Der Vakuummotor ist dann besonders einfach aufgebaut. Es wird jedoch ein körperliches Rückstellelement bevorzugt, um eine stärkere Kraft auf den Arbeitskolben ausüben zu können und die Funktion des Vakuummotors von der Orientierung zur Erdoberfläche unabhängig zu machen.

Bevorzugt ist das Mitnehmerelement über eine Stange oder einen Mitnehmerstift mit dem Arbeitskolben (oder auch Arbeitszylinder) verbunden. Besonders bevorzugt sind der Mitnehmerstift, beziehungsweise die Stange, der Mitnehmer und der Arbeitskolben einteilig ausgeführt.

Der Steuerkolben bildet mit der Gaseinlassöffnung und der Gasauslassöffnung ein Ventilelement, wobei durch die Bewegung des Steuerkolbens die Gaseinlassöffnung und die Gasauslassöffnung wiederholt geöffnet und verschlossen werden.

Mit der Erfindung wird vorgeschlagen, dass der Innenraum zwischen dem Arbeitskolben und der Rückseite bis auf die Gaseinlassöffnung und die Gasauslassöffnung geschlossen ist.

Genauer kann vorgesehen sein, dass der Innenraum zwischen dem Arbeitskolben in seiner maximal in Richtung der Vorderseite des Innenraums ausgelenkten Position und der Rückseite des Innenraums bis auf die Gaseinlassöffnung und die Gasauslassöffnung geschlossen ist.

Erfindungsgemäß kann vorgesehen sein, dass das Mitnehmerelement bei einer Bewegung des Arbeitskolbens vom Steuerkolben weg den Steuerkolben in Richtung des Arbeitskolbens nachzieht und den Steuerkolben in die erste Position überführt und das Mitnehmerelement oder der Abstandhalter bei einer Bewegung des Arbeitskolbens zum Steuerkolben hin den Steuerkolben in Richtung der Rückseite drückt und den Steuerkolben in die zweite Position überführt.

Bevorzugt ist bei erfindungsgemäßen Vakuummotoren vorgesehen, dass der Arbeitskolben und der Steuerkolben entlang der gleichen Achse linear beweglich sind. Bevorzugt kann dabei vorgesehen sein, dass der Arbeitskolben und der Steuerkolben in Richtung der Vorderseite und in Richtung der Rückseite des Innenraums linear beweglich sind.

Bei einem erfindungsgemäßen Vakuummotor ist erfindungsgemäß vorgesehen, dass der Steuerkolben in einer dritten Position zwischen der ersten Position und der zweiten Position sowohl die Gaseinlassöffnung als auch die Gasauslassöffnung abdeckt.

Hierdurch wird sichergestellt, dass der Vakuummotor nicht in einer Zwischenposition, in der die Gaseinlassöffnung und die Gasauslassöffnung gleichzeitig offen sind, stehenbleibt und durch einen solchen "Kurzschluss" stehenbleiben kann. Die Trägheit des Arbeitskolbens und/oder die Kraft des Rückstellelements sorgen dafür, dass diese beiden Öffnungen nicht dauerhaft verschlossen bleiben.

Es kann auch vorgesehen sein, dass an der dem Arbeitskolben zugewandten Seite des Steuerkolbens zumindest ein Abstandhalter angeordnet ist und/oder an der dem Steuerkolben zugewandten Seite des Arbeitskolbens oder am Mitnehmer, insbesondre am Mitnehmerstift, zumindest ein Abstandhalter angeordnet ist, so dass zwischen dem Arbeitskolben und dem Steuerkolben ein Zwischenraum entsteht, wenn diese aneinander anliegen. Mit einer Weiterentwicklung der vorliegenden Erfindung kann dabei vorgesehen sein, dass die Gaseinlassöffnung in der zweiten Position in diesen Zwischenraum mündet, wobei durch den zumindest einen Abstandhalter bei aneinander anliegenden Arbeitskolben und Steuerkolben ein zweiter Abstand zwischen dem Arbeitskolben und dem Steuerkolben eingestellt wird, der kleiner ist als ein erster Abstand, der durch das Mitnehmerelement eingestellt wird.

Der Abstandshalter kann durch ein Rohr entlang der Mittelachse des Innenraums gebildet sein, durch das sich der Mitnehmer erstreckt, beziehungsweise der Mitnehmerstift oder die Stange erstreckt, an dem oder an der der Mitnehmer am Arbeitskolben oder am Steuerkolben befestigt ist. Alternativ kann der zumindest eine Abstandshalter auch als Stift ausgebildet sein. Bevorzugt ist der Abstandhalter oder sind die Abstandhalter einteilig mit dem Arbeitskolben oder dem Steuerkolben ausgebildet.

Es ist für die Erfindung wesentlich, dass sich der Abstand zwischen dem Arbeitskolben und dem Steuerkolben vergrößert, wenn der Arbeitskolben durch das Rückstellelement in Richtung von der Rückseite weg beschleunigt wird. Dadurch wird erreicht, dass über das Mitnehmerelement ein Stoß oder eine größere Kraft zur Beschleunigung des Steuerkolbens aufgebracht werden kann, so dass der Vakuummotor nicht durch einen ruhenden oder blockierenden Steuerkolben stillstehen kann. Zu dem gleichen Zweck kann erfindungsgemäß auch vorgesehen sein, dass der Arbeitskolben, und insbesondere der Arbeitskolben mit dem fest daran verbundenen Mitnehmerelement, eine größere Masse aufweist als der Steuerkolben.

Des Weiteren kann vorgesehen sein, dass das Rückstellelement eine elastische Druckfeder ist, die im Innenraum zwischen dem Arbeitskolben und der Rückseite des Innenraums angeordnet ist.

Hierdurch wird ein besonders einfach und kostengünstig aufzubauender Vakuummotor bereitgestellt, der unabhängig von seiner Orientierung gut funktioniert. Zudem kann die Frequenz des Motors durch Wahl der Federkonstante eingestellt werden.

Dabei kann vorgesehen sein, dass die Feder am Arbeitskolben anliegt. Die Feder liegt jedoch bevorzugt nicht an der Rückseite des Innenraums an. Stattdessen liegt die Feder an einer Auflagefläche an, die zwischen der Rückseite des Innenraums und dem Arbeitskolben angeordnet ist, bevorzugt zwischen dem Steuerkolben und dem Arbeitskolben angeordnet ist. Die Auflagefläche kann durch eine Begrenzungswand eines mittleren oder vorderen Teils des Innenraums bereitgestellt sein, in dem sich der Arbeitskolben bewegt. Die Feder hat erfindungsgemäß bevorzugt eine Federkonstante zwischen 1 N/cm und 100 N/cm. Bevorzugt ist die elastische Druckfeder eine Spiralfeder oder eine Blattfeder.

Um den Vakuummotor bezogen auf die Erdoberfläche variabel positionieren zu können, wie dies beispielsweise bei handgehaltenen Lavage-Systemen erforderlich ist, und/oder um ein stärkeres Moment auf den Arbeitskolben ausüben zu können kann jedoch erfindungsgemäß bevorzugt vorgesehen sein, dass das Rückstellelement ein körperliches Rückstellelement ist, insbesondere eine elastische Feder, wie zum Beispiel eine Stahlfeder und/oder eine Gasfederung ist. Die elastische Feder kann theoretisch auch eine Zugfeder sein. Als Zugfeder ist die elastische Feder zwischen der Vorderseite des Innenraums und dem Arbeitskolben im Innenraum angeordnet. Dazu muss die Zugfeder sowohl an der Vorderseite des Innenraums als auch an dem Arbeitskolben verankert sein. Bevorzugt wird jedoch eine Druckfeder. Als Druckfeder ist die elastische Feder zwischen dem Arbeitskolben und der Rückseite des Innenraums in dem Innenraum angeordnet.

Ferner wird mit der Erfindung vorgeschlagen, dass der Steuerkolben in der ersten Position von dem Rückstellelement über das Mitnehmerelement in Richtung der Vorderseite gezogen ist, wobei die Gasauslassöffnung in den Zwischenraum zwischen dem Steuerkolben und der Rückseite des Innenraums mündet.

Durch diesen Aufbau gelingt ist, dass der Vakuummotor in einer Situation, in der kein Gas durch die Gasauslassöffnung aus dem Innenraum gezogen wird, in eine Startposition (die Ausgangsposition des Arbeitskolbens und des Steuerkolbens) gebracht wird, aus der er bei Absaugen eines Gases durch die Gasauslassöffnung startet.

Es kann auch vorgesehen sein, dass in dem Steuerkolben wenigstens ein gasdurchlässiger Durchgang angeordnet ist, die die dem Arbeitskolben zugewandte Vorderseite des Steuerkolbens mit der der Rückseite des Innenraums zugewandten Rückseite des Steuerkolbens gasdurchlässig verbindet.

Der Steuerkolben kann dann vollumfänglich an den Innenwänden des Innenraums anliegen. Dadurch wird eine stabile lineare Führung des Steuerkolbens im Innenraum gewährleistet.

Gemäß einer bevorzugten Ausgestaltung der Erfindung kann auch vorgesehen sein, dass der Arbeitskolben vollumfänglich an der Innenwand des Innenraums anliegt, bevorzugt über ein Dichtungselement vollumfänglich, gasdicht und druckdicht an dem Innenraum anliegt.

Der Arbeitskolben kann also erfindungsgemäß bevorzugt dicht gegen die Innenwände des Innenraums gelagert sein. Hierdurch wird gewährleistet, dass das komprimierte Gas nicht an dem Arbeitskolben vorbei strömt und dadurch die Funktionstüchtigkeit oder Leistung des Vakuummotors gemindert wird.

Für erfindungsgemäße Vakuummotoren zur Erzeugung einer mechanischen Bewegung kann bevorzugt vorgesehen sein, dass an der dem Steuerkolben abgewandten Seite des Arbeitskolbens eine Stange, insbesondere eine Pleuelstange befestigt ist, die aus dem Innenraum herausragt, bevorzugt durch die Vorderseite des Innenraums herausragt.

Bei dieser Variante wird durch die Stange ein periodischer Stoß erzeugt. Bei Verwendung einer Pleuelstange, die über eine Achse mit dem Arbeitskolben verbunden ist, kann die periodische Bewegung des Arbeitskolbens in eine Drehbewegung umgesetzt werden. Dazu ist die Pleuelstange bevorzugt mit einer Kurbelwelle verbunden oder verbindbar.

Bevorzugte Ausführungsformen der Erfindung können sich auch dadurch auszeichnen, dass in der der Rückseite gegenüberliegenden Vorderseite des Innenraums eine Ausstoßöffnung vorgesehen ist und in der Vorderseite und/oder in der seitlichen Wandung des Innenraums eine Flüssigkeitszufuhröffnung angeordnet ist, die zumindest zeitweise nicht vom Arbeitskolben oder einem Pumpkolben abgedeckt ist und die im nicht abgedeckten Zustand zwischen dem Arbeitskolben oder dem Pumpkolben und der Vorderseite des Innenraums angeordnet ist.

Durch diesen Aufbau wird der Hubraum des Arbeitskolbens zumindest teilweise zur Flüssigkeitsaufnahme beziehungsweise zur Aufnahme eines Gemischs aus Gas und Flüssigkeit genutzt. Wenn der Arbeitskolben in Richtung der Vorderseite des Innenraums bewegt wird, wird die Flüssigkeit, beziehungsweise das Flüssigkeits-Gas-Gemisch durch die Ausstoßöffnung ausgestoßen. Der Vakuummotor ist dann unmittelbar für Lavage-Systeme einsetzbar.

Dabei kann vorgesehen sein, dass die Ausstoßöffnung durch ein Ventilelement, insbesondere ein Lippenventil mit der Umgebung verbunden ist, wobei das Ventilelement bei einem ausreichenden Überdruck gegenüber dem Umgebungsdruck geöffnet ist und ansonsten geschlossen ist, und dass an der Flüssigkeitszufuhröffnung ein Rohr oder eine Schlauch mit einem Rückschlagventil angeschlossen ist, das sich bei einem Unterdruck im Innenraum zwischen dem Arbeitskolben oder dem Pumpkolben und der Vorderseite des Innenraums öffnet und eine Flüssigkeitszufuhr in den Innenraum ermöglicht.

Hierdurch wird eine automatische Befüllung des Innenraums mit einer Flüssigkeit erreicht. Dadurch wird ein besonders einfacher und kostengünstiger Aufbau eines Vakuummotors für ein Lavage-System erreicht. Zur Definition eines ausreichenden Drucks sei auf bekannte Lippenventile zu ähnlichen oder gleichen Zwecken verwiesen.

Ferner kann bei solchen Vakuummotoren vorgesehen sein, dass der Arbeitskolben an der der Vorderseite zugewandten Seite einen Pumpkolben aufweist, der eine zumindest 50% geringere Querschnittsfläche hat als der der Rückseite zugewandte Teil des Arbeitskolbens, wobei der Querschnitt des Innenraums an den Querschnitt des Pumpkolbens und den Querschnitt des Arbeitskolbens angepasst ist.

Hierdurch wird sichergestellt, dass der der Rückseite zugewandte Teil des Arbeitskolbens im Innenraum beweglich ist und der Pumpkolben durch die Bewegung des Arbeitskolbens die Flüssigkeitszufuhröffnung periodisch öffnen und schließen kann und einen periodischen Ausstoß von Flüssigkeit aus der Ausstoßöffnung ermöglicht.

Erfindungsgemäß bevorzugt hat der Pumpkolben eine zumindest 75% geringere Querschnittsfläche als der Arbeitskolben. Der Durchmesser ist bei zylindrischer Geometrie des Pumpkolbens und des Arbeitskolbens dann 50% geringer.

Es kann bei erfindungsgemäßen Vakuummotoren auch vorgesehen sein, dass das Mitnehmerelement eine Schnur, ein Kabel, ein Faden, eine Kette oder eine elastische Feder ist, die am Arbeitskolben und am Steuerkolben befestigt ist oder das Mitnehmerelement eine Stange, eine Schnur, ein Kabel, ein Faden, eine Kette oder eine elastische Feder aufweist, die am Arbeitskolben oder am Steuerkolben befestigt ist und an der ein Mitnehmer befestigt ist, der bei der periodischen Bewegung des Arbeitskolbens in einen Vorsprung im Arbeitskolben oder im Steuerkolben greift, wobei bevorzugt das Mitnehmerelement durch eine am Arbeitskolben befestigte Stange gebildet wird, die durch eine Durchführung im Steuerkolben reicht und an der der Mitnehmer befestigt ist, der nicht durch die Durchführung im Steuerkolben passt und der auf der Rückseite der Durchführung des Steuerkolbens in diese eingreift um den Steuerkolben mitzuziehen, wenn der Arbeitskolben dazu ausreichend weit von dem Steuerkolben entfernt ist und sich in Richtung von diesem weg bewegt.

Mit den genannten Mitnehmerelementen kann auf baulich einfache Weise eine Vergrößerung des Abstands zwischen dem Arbeitskolben und dem Steuerkolben erreicht werden, bevor der Steuerkolben von dem Arbeitskolben mitgezogen wird. Dadurch kann der Wirkungsgrad des Motors verbessert und die Gefahr eines Stillstands des Motors verringert werden.

Es kann erfindungsgemäß bevorzugt vorgesehen sein, dass der Mitnehmer auf einen gegenüberliegenden Anschlag im Arbeitskolben oder im Steuerkolben stößt, wobei der Mitnehmer auf den Anschlag im Inneren des Steuerkolbens trifft, wenn der Arbeitskolben sich in Richtung des Steuerkolbens bewegt und dicht genug an dem Steuerkolben positioniert ist.

Bei bevorzugten Ausgestaltungen der Erfindung kann auch vorgesehen sein, dass der Innenraum zumindest bereichsweise zylindrisch ist oder im Bereich eines Arbeitsraums des Arbeitskolbens oder im gesamten Hubraum des Arbeitskolbens und des Steuerkolbens zylindrisch ist.

Der zylindrische Innenraum muss nicht vollständig zylindrisch zu sein. Es reicht aus, wenn der Hubraum des Arbeitskolbens zylindrisch ist. Bevorzugt ist auch der Hubraum des Steuerkolbens zylindrisch. Der Hubraum des Steuerkolbens muss nicht zylindrisch sein, wenn darin Fräsungen oder andere Ausnehmungen vorgesehen sind, die dazu dienen, dass das Gas oder die Luft am Steuerkolben vorbei in den Raum zwischen dem Steuerkolben und der Rückseite des Innenraums gelangen kann. Der Raum zwischen der maximalen Auslenkung des Arbeitskolbens in Richtung des vorderen Endes des Innenraums und dem vorderen Ende des Innenraums muss nicht zwingend zylindrisch sein, wenn dort kein Pumpkolben vorgesehen ist. Der Aufbau des Vakuummotors wird jedoch vereinfacht, wenn auch dieser Teil des Innenraums zylindrisch ist. Ein Zylinder im Sinne der vorliegenden Erfindung ist, wie auch nach der allgemeinen Definition, ein von zwei parallelen, ebenen, kongruenten Flächen (Grund- und Deckfläche) und einer Mantelfläche beziehungsweise Zylinderfläche begrenzter Körper, wobei die Mantelfläche von parallelen Geraden gebildet wird. Das heißt, der Zylinder entsteht durch Verschiebung einer ebenen Fläche entlang einer Geraden, die nicht in dieser Ebene liegt. Die Höhe des Zylinders ist gegeben durch den Abstand der beiden Ebenen, in denen Grund- und Deckfläche liegen.

Sind die Geraden senkrecht zu Grund- und Deckfläche, spricht man von einem geraden Zylinder. Die gerade zylindrische Geometrie des Innenraums ist erfindungsgemäß bevorzugt, bezieht sich aber besonders bevorzugt nur auf einen oder mehrere Teilbereich des gesamten Innenraums. Ein gerader Kreiszylinder stellt im Sinne der vorliegenden Erfindung also nur einen Spezialfall einer zylindrischen Geometrie dar.

Der Arbeitskolben schließt in allen Positionen mit den Innenwänden des Innenraums dicht ab, so dass der Innenraum stets durch den Arbeitskolben in zumindest zwei voneinander getrennte Kammern getrennt ist. Bevorzugt ist der Innenraum durch den Arbeitskolben und den Pumpkolben in drei voneinander getrennte Kammern getrennt. Die Trennung erfolgt bevorzugt gasdicht und druckdicht.

Erfindungsgemäß kann vorgesehen sein, dass der Steuerkolben in axialer Richtung des Innenraums mindestens so lang ist wie die Summe des axialen Abstands der Gaseinlassöffnung von der Gasauslassöffnung und der axialen Querschnitte der Gaseinlassöffnung und der Gasauslassöffnung. Die axialen Querschnitte der Gaseinlassöffnung und der Gasauslassöffnung sind die Querschnitte der Gaseinlassöffnung und der Gasauslassöffnung in axialer Richtung des Innenraums.

Bevorzugt erfolgt die lineare Bewegung des Arbeitskolbens und des Steuerkolbens entlang einer Geraden, die der Symmetrieachse des zylindrischen Innenraums entspricht.

Gemäß einer bevorzugten Variante der vorliegenden Erfindung kann vorgesehen sein, dass der Arbeitskolben zwei unterschiedlich große Querschnittsflächen senkrecht zur linearen Bewegungsrichtung des Arbeitskolbens aufweist, wobei der Innenraum dazu passende Innenwände mit unterschiedlichen Querschnittsflächen aufweist und die Querschnittsfläche auf der der Rückseite zugewandten Seite des Arbeitskolbens zumindest 100% größer ist als die Querschnittsfläche der gegenüberliegenden Vorderseite des Arbeitskolbens, bevorzugt die Querschnittsfläche auf der, der Rückseite zugewandten Seite des Arbeitskolbens zumindest vier Mal so groß ist wie die Querschnittsfläche der gegenüberliegenden Vorderseite des Arbeitskolbens. Der vordere Teil des Arbeitskolbens wird vorliegend meist als Pumpkolben bezeichnet.

Dies hat den Vorteil, dass durch den größeren Querschnitt des der Rückseite zugewandten Teils des Arbeitskolbens, an dem das durch den Vakuummotor strömende Gas die Arbeit leistet, eine größere Kraft wirken kann, um ein Rückstellelement mit einer größeren Rückstellkraft zu spannen, was wiederum zu einer Erhöhung der Arbeitsfrequenz des Vakuummotors führt.

Es kann erfindungsgemäß bevorzugt vorgesehen sein, dass der, der Rückseite zugewandte Teil des Arbeitskolbens oder der Arbeitskolben einen Durchmesser von zumindest 4 cm aufweist.

Bei ovalen oder kreisförmigen Querschnittsflächen des Arbeitskolbens kann anstatt von einer Querschnittsfläche immer auch von einem Radius, Durchmesser oder Querschnitt gesprochen werden. Dementsprechend kann bei ovalen oder kreisförmigen Querschnittsflächen des Arbeitskolbens oder bei anderen Querschnittsflächen des Arbeitskolbens, auf die ein Querschnitt, Radius oder Durchmesser anwendbar ist, erfindungsgemäß vorgesehen sein, dass der Arbeitskolben zwei unterschiedlich große Durchmesser oder Querschnitte senkrecht zur Bewegungsrichtung des Arbeitskolbens aufweist, wobei der Innenraum dazu passende Innenwände mit unterschiedlichen Durchmessern oder Querschnitten aufweist und der Durchmesser oder der Querschnitt auf der, der Rückseite zugewandten Seite des Arbeitskolbens größer ist als der Durchmesser oder der Querschnitt der gegenüberliegenden Vorderseite des Arbeitskolbens. Bevorzugt kann dabei vorgesehen sein, dass der Durchmesser oder der Querschnitt auf der, der Rückseite zugewandten Seite des Arbeitskolbens mindestens doppelt so groß wie der Durchmesser oder der Querschnitt der gegenüberliegenden Vorderseite des Arbeitskolbens ist.

Mit einer Weiterbildung der Erfindung wird vorgeschlagen, dass in dem Innenraum ein zweites Rückstellelement angeordnet ist, das bei laufendem Vakuummotor zumindest zeitweise auf den Steuerkolben eine Kraft in Richtung des Arbeitskolbens ausübt, wobei bevorzugt zwischen dem Steuerkolben und der Rückseite des Innenraums das zweite elastische Rückstellelement angeordnet ist, besonders bevorzugt eine elastische Druckfeder als zweites Rückstellelement zwischen dem Steuerkolben und der Rückseite des Innenraums angeordnet ist.

Hierdurch kann eine Positionierung des Steuerkolbens erreicht werden, bei der im Ruhezustand des Vakuummotors die Gasauslassöffnung freiliegt, also nicht von dem Steuerkolben abgedeckt wird.

Erfindungsgemäß kann ferner vorgesehen sein, dass der Vakuummotor aus thermoplastischen Kunststoffen gefertigt ist, wobei Polypropylen, Polyethylen, Polyamid-6 und Polyamid-12 besonders bevorzugt sind. Daneben sind alle technisch üblichen Kunststoffe geeignet.

Erfindungsgemäße Vakuummotoren arbeiten bevorzugt mit einer Frequenz von 500 bis 2000 Zyklen pro Minute, besonders bevorzugt mit einer Frequenz von 1000 bis 1500 Zyklen pro Minute.

Die Aufgaben der Erfindung bezüglich eines Lavage-Systems werden gelöst durch ein Lavage-System aufweisend zumindest einen Vakuummotor nach einem der vorangehenden Ansprüche, bei dem mit dem Vakuummotor oder den Vakuummotoren ein periodischer Sprühstoß mit einer Flüssigkeit erzeugbar ist.

Bei dem Lavage-System kann vorgesehen sein, dass das Lavage-System einen Anschluss für eine Unterdruckquelle und ein manuell betätigbares Steuerungsventil aufweist, wobei das Steuerungsventil über eine Gasleitung mit der Unterdruckquelle verbunden ist und die Gasauslassöffnung des Vakuummotors über eine Gasleitung mit dem Steuerungsventil verbunden ist. Erfindungsgemäß bevorzugt kann vorgesehen sein, dass eine Flüssigkeitszufuhröffnung des Vakuummotors mit einer Flüssigkeitsleitung verbunden ist.

Die Aufgaben der Erfindung werden ferner gelöst durch die Verwendung eines erfindungsgemäßen Vakuummotors als Motor für einen Klopfermotor, einen Vibrationsmotor, als Antrieb für eine Dosiervorrichtung, als Rüttelmotor oder als Pumpe, insbesondere als Schmiermittelpumpe.

Die der Erfindung zugrundeliegenden Aufgaben werden auch gelöst durch ein Verfahren zum Erzeugen einer periodischen Bewegung mit einem Vakuum oder einem Unterdruck, insbesondere unter Verwendung eines erfindungsgemäßen Vakuummotors, bei dem
A) in einem Ausgangszustand ein Arbeitskolben und ein Steuerkolben in einem Innenraum voneinander mit einem ersten Abstand beabstandet werden, wobei der Steuerkolben eine Gaseinlassöffnung verschließt und eine Gasauslassöffnung offen liegt;
B) das Gas zwischen dem Arbeitskolben und dem Steuerkolben und zwischen dem Arbeitskolben und einer Rückseite des Innenraums durch die Gasauslassöffnung aus dem Innenraum herausgezogen wird;
C) der Arbeitskolben durch die Druckdifferenz zwischen dem auf der Vorderseite und der Rückseite des Arbeitskolbens lastenden Gasdruck in Richtung der Rückseite des Innenraums zum Steuerkolben hin beschleunigt und bewegt wird, wobei sich der Abstand zwischen dem Arbeitskolben und dem Steuerkolben verringert und das Rückstellelement durch die Bewegung des Arbeitskolbens Energie aufnimmt und speichert;
D) der Steuerkolben über ein Mitnehmerelement oder einen Abstandhalter von dem Arbeitskolben mit geschoben wird, bevorzugt ab Erreichen eines zweiten Abstands;
E) der Steuerkolben die Gasauslassöffnung aufgrund der Bewegung des Steuerkolbens verschließt;
F) der Steuerkolben die Gaseinlassöffnung aufgrund der Bewegung des Steuerkolbens öffnet;
G) das ein Druckgas oder die umgebende Luft durch die Gaseinlassöffnung in den Innenraum strömt, bevorzugt durch die Gaseinlassöffnung zwischen dem Arbeitskolben und dem Steuerkolben in den Innenraum strömt;
H) der Arbeitskolben durch das Rückstellelement in Richtung einer Vorderseite des Innenraums beschleunigt wird;
I) der Steuerkolben über das Mitnehmerelement von dem Arbeitskolben mitgezogen wird und diesen in Richtung der Vorderseite des Innenraums bewegt, bevorzugt ab Erreichen eines ersten Abstands;
J) durch die umgekehrte Bewegung des Steuerkolbens die Gaseinlassöffnung wieder geschlossen wird und
K) durch die umgekehrte Bewegung des Steuerkolbens die Gasauslassöffnung wieder geöffnet wird, so dass erneut das Gas zwischen dem Arbeitskolben und der Rückseite des Innenraums aus dem Innenraum heraus gezogen wird.

Die Schritte finden in der logischen und chronologischen Reihenfolge statt, wobei sich die Zeiträume, zu denen die erfindungsgemäßen Schritte stattfinden, teilweise und zeitweise überlagern. Bevorzugt wird der Vakuummotor durch das Rückstellelement wieder in den Ausgangszustand zurückgeführt. Der Vakuummotor funktioniert jedoch auch, wenn der Ausgangszustand erst wieder erreicht wird, wenn kein Unterdruck mehr an der Gasauslassöffnung zur Verfügung steht.

Bei erfindungsgemäßen Verfahren kann vorgesehen sein, dass der Zyklus sich mit dem erneuten Evakuieren des Gases im Innenraum zwischen dem Arbeitskolben und der Rückseite des Innenraums wiederholt.

Ferner kann erfindungsgemäß vorgesehen sein, dass der zweite Abstand zwischen dem Arbeitskolben und dem Steuerkolben mit Hilfe zumindest eines Abstandhalters oder des Mitnehmerelements eingestellt wird und der erste Abstand zwischen dem Arbeitskolben und dem Steuerkolben mit dem Mitnehmerelement eingestellt wird, vorzugsweise durch die Länge des Mitnehmerelements.

Bevorzugt wird der erste Abstand zumindest 1,5-mal so groß wie der zweite Abstand gewählt.

Es kann bei erfindungsgemäßen Verfahren ferner vorgesehen sein, dass die periodische, lineare Bewegung des Arbeitskolbens im Vakuummotor selbständig durch Einwirkung von Vakuum oder Unterdruck ohne Einwirkung äußerer Ventile ausgelöst wird.

Es wird erfindungsgemäß bevorzugt, dass das Vakuum oder der Unterdruck einen Unterdruck gegenüber der umgebenden Atmosphäre von wenigstens 0,5 bar hat.

Erfindungsgemäße Verfahren werden bevorzugt mit einer Frequenz von 500 bis 2000 Zyklen pro Minute wiederholt, besonders bevorzugt mit einer Frequenz von 1000 bis 1500 Zyklen pro Minute wiederholt.

Die der Erfindung zugrundeliegenden Aufgaben werden auch gelöst durch ein Verfahren zum Erzeugen eines Sprühstoßes aufweisend die genannten erfindungsgemäßen Verfahrensschritte, wobei bei einer Bewegung des Arbeitskolbens, insbesondere eines Pumpkolbens des Arbeitskolbens, weg von der Rückseite des Innenraums eine Spülflüssigkeit oder ein Flüssigkeit-Gas-Gemisch aus dem Raum zwischen dem Arbeitskolben und der Vorderseite des Innenraums durch eine Ausstoßöffnung an der Vorderseite des Innenraums hinaus gepresst wird und bei einer Bewegung des Arbeitskolbens, insbesondere des Pumpkolbens des Arbeitskolbens, auf die Rückseite des Innenraums zu eine Flüssigkeit oder ein Flüssigkeit-Gas-Gemisch in den Raum zwischen dem Arbeitskolben, insbesondere dem Pumpkolben, und der Vorderseite des Innenraums durch eine Flüssigkeitszufuhröffnung hinein gedrückt oder gezogen wird.

Bei diesem Verfahren kann vorgesehen sein, dass bei der Bewegung des Arbeitskolbens, insbesondere des Pumpkolbens, auf die Vorderseite des Innenraums zu durch den Druck in dem Raum zwischen dem Arbeitskolben, insbesondere dem Pumpkolben, und der Vorderseite des Innenraums ein Ventil an der Ausstoßöffnung geöffnet wird und/oder offen gehalten wird und ein an die Flüssigkeitszufuhröffnung angeschlossenes Rückschlagventil geschlossen wird und/oder geschlossen gehalten wird und bei der Bewegung des Arbeitskolbens, insbesondere des Pumpkolbens, auf die Rückseite des Innenraums zu durch den geringeren Druck in dem Raum zwischen dem Arbeitskolben, insbesondere dem Pumpkolben, und der Vorderseite des Innenraums das Ventil an der Ausstoßöffnung geschlossen wird und/oder geschlossen gehalten wird und das an die Flüssigkeitszufuhröffnung angeschlossene Rückschlagventil geöffnet wird und/oder offen gehalten wird.

Der Erfindung liegt die überraschende Erkenntnis zugrunde, dass es durch die im Abstand zueinander veränderlichen Arbeitskolben und Steuerkolben gelingt, einen einfachen aber gleichzeitig zuverlässig laufenden Vakuummotor bereitzustellen, der keinen Totpunkt aufweist, an dem der Vakuummotor zum Stehen kommt, ohne dass er wieder anlaufen würde. Der "tote Punkt" wird erfindungsgemäß dadurch vermieden, dass bei dem Vakuummotor immer ein definierter Zustand der Ventile, also des Steuerkolbens und der Abdeckung der Gaseinlassöffnung und der Gasauslassöffnung durch den Steuerkolben möglich ist. Unter definiertem Zustand ist zu verstehen, dass ein Ventil entweder geöffnet oder geschlossen ist. Dieser definierte Zustand wird durch die von dem Rückstellelement erzeugte Kraft auf den Arbeitskolben sichergestellt. Durch eine geeignete Einstellung der beiden Abstände der beiden Kolben (Arbeitskolben und Steuerkolben) zueinander während des Betriebs des Vakuummotors kann sichergestellt werden, dass der Vakuummotor passend zu dem jeweiligen Zweck einsetzbar ist. Insbesondere als Pumpe zum Erzeugen von Sprühstößen einer Spülflüssigkeit für eine Spülvorrichtung kann der Vakuummotor auf einfachste Weise ausgelegt werden, indem der Hubraum des Arbeitskolbens unmittelbar als Pumpe verwendet wird, wobei bevorzugt der vordere Teil des Arbeitskolbens, der im Rahmen der vorliegenden Erfindung auch als Pumpkolben bezeichnet wird und der einen geringeren Hubraum aufweist als der hintere Teil des Arbeitskolbens, an dem die Arbeit durch das Gas geleistet wird. Der geringere Hubraum wird durch einen geringeren Querschnitt senkrecht zur Bewegungsrichtung, insbesondere der Zylinderachse des Arbeitskolbens erreicht.

Der Vakuummotor kann die in vielen Bereichen, insbesondere in Operationssälen, ohnehin vorhandenen Unterdruckquellen beziehungsweise Vakuumquellen nutzen, um den Vakuummotor beziehungsweise den Arbeitskolben anzutreiben. Es wird dann keine zusätzliche Energiequelle zum Antreiben des Vakuummotors benötigt. Gleichzeitig ist in solchen Fällen der Unterdruck beziehungsweise das Vakuum in praktisch unbegrenzter Menge vorhanden.

Der Arbeitskolben und der Steuerkolben bewegen sich dabei nicht gleichzeitig mit konstantem Abstand zueinander. Zunächst bewegt sich der Arbeitskolben. Wenn der Arbeitskolben eine gewisse Entfernung zum Steuerkolben unterschreitet und dadurch eine gewisse Geschwindigkeit erreicht hat, drückt dieser den Steuerkolben ruckartig in Richtung der Rückseite des Innenraums des Vakuummotors, in dem der Mitnehmer auf den Vorsprung (zweiten Anschlag) des Steuerkolbens oder des Arbeitskolbens schlägt oder der Abstandhalter des Arbeitskolbens auf den Steuerkolben trifft oder der Abstandhalten des Steuerkolbens auf den Arbeitskolben trifft und dadurch in allen diesen Fällen der Steuerkolben mitbewegt wird. Die der vorliegenden Erfindung zugrundeliegende Idee besteht also insbesondere darin, eine zeitliche Entkopplung der Ventilsteuerung durch den Steuerkolben und der Bewegung des Arbeitskolbens zu erreichen. Hierdurch kann auf überraschende Weise eine kraftvolle Bewegung des Arbeitskolbens erreicht werden. Auch im Vergleich zu dem Druckgasmotor nach der WO 2012/038003 A1 mit dem zweiteiligen aber fest verbundenen Arbeitskolben ergibt sich mit dem erfindungsgemäßen Vakuummotor eine kräftiger Bewegung des Arbeitskolbens, bei der also mit jeder Bewegung des Arbeitskolbens mehr Kraft übertragen werden kann.

Dadurch ist es möglich, den vorderen Teil des Innenraums unmittelbar und ohne ein Getriebe oder eine Übersetzung zur Kraftverstärkung als Pumpraum einzusetzen. Der Sprühstoß beziehungsweise der Spülflüssigkeitsstoß eines Lavage-Systems kann so unmittelbar mit Hilfe des Arbeitskolbens erzeugt werden. Dadurch lassen sich Energieverluste beim Pumpen vermeiden. Ein Defekt des Getriebes oder eines Teils der Übersetzung ist hierdurch ausgeschlossen. Zudem wird der Aufbau kompakter und kostengünstiger, als wenn solche Teile angebaut oder eingebaut werden müssten.

Die Erfindung basiert dabei auf folgender Idee und kann wie folgt ausgeführt werden: Ein axial verschiebbarer Arbeitskolben wird in einem Hohlzylinder mit zwei oder drei verschiedenen Durchmesserbereichen durch ein Rückstellelement an eine Seite des Hohlzylinders oder in Richtung einer Seite des Hohlzylinders gedrückt. In der Mantelfläche des Hohlzylinders sind eine Gaseinlassöffnung und eine Gasauslassöffnung getrennt voneinander angeordnet, die gasdurchlässig mit dem Innenraum des Hohlzylinders verbunden sind. Die Gaseinlassöffnung befindet sich in Richtung des Arbeitskolbens und des Rückstellelements und die Gasauslassöffnung ist entgegengesetzt dazu in Richtung des gasundurchlässigen Verschlusses des Hohlzylinders angeordnet. Ein Steuerkolben beziehungsweise ein Ventilelement ist axial im Hohlzylinder verschiebbar und kann je nach Position entweder nur die Gaseinlassöffnung verschließen, wobei gleichzeitig die Gasauslassöffnung geöffnet ist oder nur die Gasauslassöffnung verschließen wobei gleichzeitig die Gaseinlassöffnung geöffnet ist.

Der Steuerkolben beziehungsweise das Ventilelement besitzt einen Hohlraum, der sich axial durch den Steuerkolben beziehungsweise das Ventilelement erstreckt. An der Stirnseite des Arbeitskolbens, an dem das Rückstellelement ansetzt, befindet sich ein Mitnehmerstift, an dessen Ende ein Mitnehmer angeordnet ist. Der Mitnehmerstift ist in dem Hohlraum des Ventilelements axial beweglich. Am Ventilelement befindet sich in Richtung der Stirnseite des Arbeitskolbens, an dem der Mitnehmerstift angeordnet ist, ein erster Anschlag und ein zweiter Anschlag ist an der anderen Schmalseite des Ventilelements angeordnet. An dem zweiten Anschlag kann der Mitnehmer bei axialer Bewegung des Mitnehmerstifts infolge der Bewegung des Arbeitskolbens in Richtung des Rückstellelements ansetzen und bei der axialen Bewegung das Ventilelement entgegengesetzt zum Rückstellelement bewegen. Dabei wird die Gasauslassöffnung geschlossen und die Gaseinlassöffnung geöffnet. Bei der entgegengesetzten Bewegung des Arbeitskolbens infolge der Einwirkung des Rückstellelements schlägt der Mitnehmer an den ersten Anschlag an und verschiebt das Ventilelement axial in Richtung des Arbeitskolbens, wobei die Gaseinlassöffnung geschlossen wird und die Gasauslassöffnung geöffnet wird.

Vorteilhat ist auf der Stirnseite des Arbeitskolbens, die der Stirnseite mit dem daran angeordneten Mitnehmerstift entgegengesetzt ist, mindestens ein Beabstandungselement beziehungsweise ein Abstandhalter angeordnet, das oder der diese Stirnseite des Arbeitskolbens von der Wand des Hohlzylinders beabstandet, wodurch ein Spalt gebildet wird. Dieser ist notwendig, um ein unerwünschtes Ansaugen des Arbeitskolbens an die Hohlzylinderwand zu verhindern.

Der Vakuummotor funktioniert in der Weise, dass zuerst Vakuum durch die geöffnete Gasauslassöffnung gezogen wird. Der Unterdruck zieht den Arbeitskolben gegen das Rückstellelement. Dieses wird dabei gespannt. Dabei bewegt sich der Mitnehmerstift mit dem Mitnehmer innerhalb des Ventilelements, das dabei noch bewegt wird. Wenn bei weiterer Bewegung des Arbeitskolbens in Richtung des Rückstellelements und damit des Mitnehmerstifts innerhalb des Ventilelements der Mitnehmer auf den zweiten Anschlag trifft, wird das Ventilelement axial verschoben. Dabei wird das Ventilelement über die Gasauslassöffnung geschoben und damit wird diese verschlossen. Die bisher durch das Ventilelement verschlossene Gaseinlassöffnung wird dabei durch Verschiebung des Ventilelements geöffnet. Dann strömt Umgebungsluft in den Hohlzylinder. Der Unterdruck wird abgebaut. Es wirkt jetzt kein oder nur ein geringer Unterdruck auf den Arbeitskolben ein. Dadurch expandiert das Rückstellelement und bewegt den Arbeitskolben zurück in seine Ausgangslage, wobei sich das Rückstellelement weitgehend entspannt. Bei der axialen Bewegung des Arbeitskolbens entgegengesetzt zum Rückstellelement wird der Mitnehmerstift ebenfalls in diese Richtung bewegt. Der Mitnehmer schlägt dann an den ersten Anschlag an und verschiebt wieder das Ventilelement, wobei die Gaseinlassöffnung verschlossen wird und die Gasauslassöffnung geöffnet wird. Dann wiederholt sich der Vorgang solange Unterdruck an der Gasauslassöffnung anliegt. Wesentlich dafür ist, dass die Bewegung des Ventilelements nicht synchron zur Bewegung des Arbeitskolbens erfolgt. Dadurch kommt es durch die Trägheit des bewegten Arbeitskolbens und des Mitnehmerstifts zu einem Überfahren der Gaseinlassöffnung und der Gasauslassöffnung durch das Ventilelement. Durch die Trägheit des bewegten Arbeitskolbens wird hierbei der sogenannte "tote Punkt" überfahren, ohne dass dazu Schwungräder oder ähnliche Vorrichtungen notwendig sind.

Das automatische Starten des Vakuummotors ist dadurch möglich, dass ohne Vakuumeinwirkung der Arbeitskolben durch Einwirkung des Rückstellelements in die vorbestimmte Ausgangslage geschoben wird, bei der die Gasauslassöffnung geöffnet ist und die Gaseinlassöffnung durch das Ventilelement geschlossen ist. Dadurch ist ein automatisches Anlaufen des Vakuummotors jederzeit gewährleistet, weil das Vakuum beziehungsweise der Unterdruck bei geöffneter Gasauslassöffnung auf den Arbeitskolben einwirken kann.

Als Ventilelement wird erfindungsgemäß der Steuerkolben verwendet, der linear beweglich im Innenraum des Vakuummotors angeordnet ist.

Ein erfindungsgemäßer Vakuummotor kann dazu beispielsweise zusammengesetzt sein aus:
a) einem Hohlzylinder, der mindestens eine Gasauslassöffnung und eine Gaseinlassöffnung aufweist, die an der Mantelfläche des Hohlzylinders getrennt voneinander angeordnet sind,
b) einem im Hohlzylinder axial beweglich angeordneten Arbeitskolben,
c) einem im Hohlzylinder angeordneten elastischen Rückstellelement, das an einer Stirnseite des Arbeitskolbens angreift,
d) einem Mitnehmerstift, der an der Stirnseite des Arbeitskolbens angeordnet ist, an der das Rückstellelement angreift,
e) einem Mitnehmer, der mit dem Mitnehmerstift verbunden ist,
f) einem axial im Hohlzylinder verschiebbaren Steuerkolben als Ventilelement, der in axialer Richtung mindestens so lang ist wie die Summe des axialen Abstands zwischen der Gasauslassöffnung und der Gaseinlassöffnung, wobei der Steuerkolben bei axialer Verschiebung über die Gasauslassöffnung die Gasauslassöffnung gasdicht verschließt und gleichzeitig die Gaseinlassöffnung geöffnet ist, der Steuerkolben bei Verschiebung über die Gaseinlassöffnung die Gaseinlassöffnung gasdicht verschließt und gleichzeitig die Gasauslassöffnung geöffnet ist,
g) der Steuerkolben mindestens einen Hohlraum besitzt, in dem der Mitnehmerstift axial verschoben werden kann,
h) einem ersten Anschlag am Steuerkolben, der an der dem Arbeitskolben zugewandten Seite des Steuerkolbens so angeordnet ist, dass bei der durch Entspannen des Rückstellelements verursachten Bewegung des Arbeitskolbens der Mitnehmerstift durch den Hohlraum des Steuerkolbens bewegt wird und dann der am Mitnehmerstift angeordnete Mitnehmer auf den ersten Anschlag trifft und den Steuerkolben bei weiterer Bewegung des Arbeitskolbens in zur Expansionsrichtung des Rückstellelements gleicher Richtung bewegt wird,
i) einem zweiten Anschlag am Steuerkolben, der an der dem Arbeitskolben abgewandten Seite des Steuerkolbens so angeordnet ist, dass bei Bewegung des Arbeitskolbens durch Vakuumeinwirkung unter Zusammenpressen des Rückstellelements der Mitnehmerstift durch den Hohlraum des Steuerkolbens bewegt wird und dann der am Mitnehmerstift angeordnete Mitnehmer auf den zweiten Anschlag trifft und den Steuerkolben bei weiterer Bewegung des Arbeitskolbens in zur Expansionsrichtung des Rückstellelements entgegengesetzter Richtung bewegt, und
j) einem gasundurchlässigen Verschluss am Ende des Holzylinders, so dass ein Hohlraum durch den Holzylinder gebildet wird, der durch den Arbeitskolben und den gasundurchlässigen Verschluss abgegrenzt wird, in dem sich der Mitnehmerstift, der Mitnehmer und der Steuerkolben befinden.

Ein erfindungsgemäßes Verfahren zur Erzeugung einer periodischen, linearen Bewegung mit einem erfindungsgemäßen Vakuummotor kann beispielsweise dadurch realisiert werden, dass
a) zuerst durch das Rückstellelement der Arbeitskolben so geschoben ist, dass das Ventilelement beziehungsweise der Steuerkolben durch Überdeckung die Gaseinlassöffnung verschließt und die Gasauslassöffnung geöffnet ist,
b) durch die offene Gasauslassöffnung durch Einwirkung von Unterdruck Gas aus dem Hohlraum gesaugt wird, wobei der Arbeitskolben gegen das Rückstellelement bewegt wird, bis der Mitnehmer den zweiten Anschlag erreicht,
c) dass dann der Mitnehmer an den zweiten Anschlag des Steuerkolbens greift und bei der weiteren Bewegung des Arbeitskolbens den Steuerkolben axial verschiebt, wodurch die Gasauslassöffnung durch den Steuerkolben verschlossen wird und die Gasauslassöffnung geöffnet wird,
d) dass dann Luft aus der umgebenden Atmosphäre durch die geöffnete Gaseinlassöffnung einströmt, bis der noch vorhandene Unterdruck so niedrig ist, dass die Rückstellkraft des Rückstellelements größer ist als die Kraft des im Hohlzylinder verbliebenen restlichen Unterdrucks, wodurch das Rückstellelement den Arbeitskolben in Richtung axial in die Ausgangsposition verschiebt, wobei der Mitnehmer an den ersten Anschlag stößt und dann den Steuerkolben axial so verschiebt, dass durch den Steuerkolben die Gaseinlassöffnung durch Überdeckung geschlossen wird und die Gasauslassöffnung geöffnet wird, und
e) dass dann der Prozess der Schritte a-d wiederholt wird.

Der Prozess wird wiederholt, so lange das Vakuum oder der Unterdruck über die Gasauslassöffnung mit dem Hohlraum beziehungsweise dem Innenraum des Vakuummotors verbunden wird.

Ein weiteres Ausführungsbeispiel für die Erfindung ist eine Unterdruck-getriebene Flüssigkeitspumpe mit einem erfindungsgemäßen Vakuummotor zusammengesetzt aus
a) einem Hohlzylinder
b) einer Gasauslassöffnung im Hohlzylinder,
c) einer Gaseinlassöffnung im Hohlzylinder, wobei die Gaseinlassöffnung getrennt von der Gasauslassöffnung angeordnet ist,
d) einer Flüssigkeitseinlassöffnung im Hohlzylinder,
e) einer Flüssigkeitsauslassöffnung im Hohlzylinder,
f) einem im Hohlzylinder axial beweglich angeordneten Arbeitskolben, der eine erste Stirnseite und eine zweite Stirnseite besitzt,
g) das der Hohlzylinder an beiden Stirnseiten geschlossen ist,
h) dass das der Hohlzylinder einen ersten Hohlraum A mit der ersten Stirnseite des Arbeitskolbens bildet, wobei die Flüssigkeitseinlassöffnung und auch die Flüssigkeitsauslassöffnung den ersten Hohlraum mit dem Flüssigkeitszulauf und dem Flüssigkeitsablauf verbinden,
i) einem im Hohlzylinder angeordneten elastischen Rückstellelement, das an einer zweite Stirnseite des Arbeitskolbens angreift,
j) einem Mitnehmerstift der an der zweiten Stirnseite des Arbeitskolbens angeordnet ist, an der das Rückstellelement angreift,
k) einem Mitnehmer der mit dem Mitnehmerstift verbunden ist,
l) einem axial im Hohlzylinder verschiebbaren Steuerkolben, der in axialer Achse mindestens so lang ist wie die Summe des axialen Abstands zwischen der Gasauslassöffnung und der Gaseinlassöffnung, wobei der Steuerkolben bei axialer Verschiebung über die Gasauslassöffnung die Gasauslassöffnung gasdicht verschließt und gleichzeitig die Gaseinlassöffnung geöffnet ist, der Steuerkolben bei Verschiebung über die Gaseinlassöffnung die Gaseinlassöffnung gasdicht verschließt und gleichzeitig die Gasauslassöffnung geöffnet ist,
m) der Steuerkolben mindestens einen Hohlraum besitzt, in dem der Mitnehmerstift axial verschoben werden kann,
n) einem ersten Anschlag am Steuerkolben, der an der dem Arbeitskolben zugewandten Seite des Steuerkolbens so angeordnet ist, dass bei der durch Entspannen des Rückstellelements verursachten Bewegung des Arbeitskolbens der Mitnehmerstift durch den Hohlraum des Steuerkolbens bewegt wird und dann der am Mitnehmerstift angeordnete Mitnehmer auf den ersten Anschlag trifft und den Steuerkolben bei weiterer Bewegung des Arbeitskolbens in zur Expansionsrichtung des Rückstellelements gleicher Richtung bewegt wird, und
o) einem zweiten Anschlag am Steuerkolben, der an der dem Arbeitskolben abgewandten Seite des Steuerkolbens so angeordnet ist, dass bei Bewegung des Arbeitskolbens durch Vakuumeinwirkung unter Zusammenpressen des Rückstellelements der Mitnehmerstift durch den Hohlraum des Steuerkolbens bewegt wird und dann der am Mitnehmerstift angeordnete Mitnehmer auf den zweiten Anschlag trifft und den Steuerkolben bei weiterer Bewegung des Arbeitskolbens in zur Expansionsrichtung des Rückstellelements entgegengesetzter Richtung bewegt,
p) einem gasundurchlässigen Verschluss am Ende des Holzylinders, so dass ein Hohlraum durch den Holzylinder gebildet wird, der durch den Arbeitskolben und den gasundurchlässigen Verschluss abgegrenzt wird, in dem sich der Mitnehmerstift, der Mitnehmer und der Steuerkolben befinden.

Im Folgenden werden weitere Ausführungsbeispiele der Erfindung anhand von elf schematisch dargestellten Figuren erläutert, ohne jedoch dabei die Erfindung zu beschränken. Dabei zeigt:
- Figur 1:: eine schematische Querschnittansicht eines erfindungsgemäßen Vakuummotors im Ausgangszustand beziehungsweise der Startposition, bei der die Gaseinlassöfifnung geschlossen ist und die Gasauslassöfifnung geöffnet ist;
- Figur 2:: eine schematische Querschnittansicht des erfindungsgemäßen Vakuummotors nach Figur 1, bei dem der Arbeitskolben durch das Vakuum oder die Druckdifferenz bewegt ist;
- Figur 3:: eine schematische Querschnittansicht des erfindungsgemäßen Vakuummotors nach Figur 1, bei dem der Mitnehmer des Arbeitskolbens auf den Steuerkolben trifft;
- Figur 4:: eine schematische Querschnittansicht des erfindungsgemäßen Vakuummotors nach Figur 1, bei dem der Steuerkolben vom Arbeitskolben derart verschoben ist, dass die Gaseinlassöffnung und die Gasauslassöffnung geschlossen sind;
- Figur 5:: eine schematische Querschnittansicht des erfindungsgemäßen Vakuummotors nach Figur 1, bei dem der Steuerkolben vom Arbeitskolben derart verschoben ist, dass die Gaseinlassöffnung geöffnet ist und die Gasauslassöffnung geschlossen ist;
- Figur 6:: eine schematische Querschnittansicht des erfindungsgemäßen Vakuummotors nach Figur 1, bei dem der Arbeitskolben durch das Rückstellelement wieder entgegengesetzt bewegt ist und den Steuerkolben vom Mitnehmer wieder in Richtung der Vorderseite gezogen wurde;
- Figur 7:: eine schematische Querschnittansicht eines alternativen erfindungsgemäßen Vakuummotors mit einem Abstandhalter am Mitnehmerstift und einem Rückschlagventil an der Flüssigkeitszufuhröffnung;
- Figur 8:: eine schematische Querschnittansicht eines weiteren alternativen erfindungsgemäßen Vakuummotors mit einem Abstandhalter am Steuerkolben und mit einer Stange zur Bildung eines stoßenden Motors;
- Figur 9:: eine schematische Querschnittansicht eines vierten alternativen erfindungsgemäßen Vakuummotors mit einer Schnur oder einem Kabel als Mitnehmerelement und einem Rückschlagventil;
- Figur 10:: eine schematische Querschnittansicht eines fünften alternativen erfindungsgemäßen Vakuummotors, bei dem das Mitnehmerelement am Steuerkolben befestigt ist; und
- Figur 11:: eine schematische Querschnittansicht eines erfindungsgemäßen Lavage-Systems mit einem erfindungsgemäßen Vakuummotor.

In den Figuren werden teilweise für gleiche oder gleichartige Bauteile die gleichen Bezugszeichen verwendet, auch wenn es sich um unterschiedliche Vakuummotoren handelt.

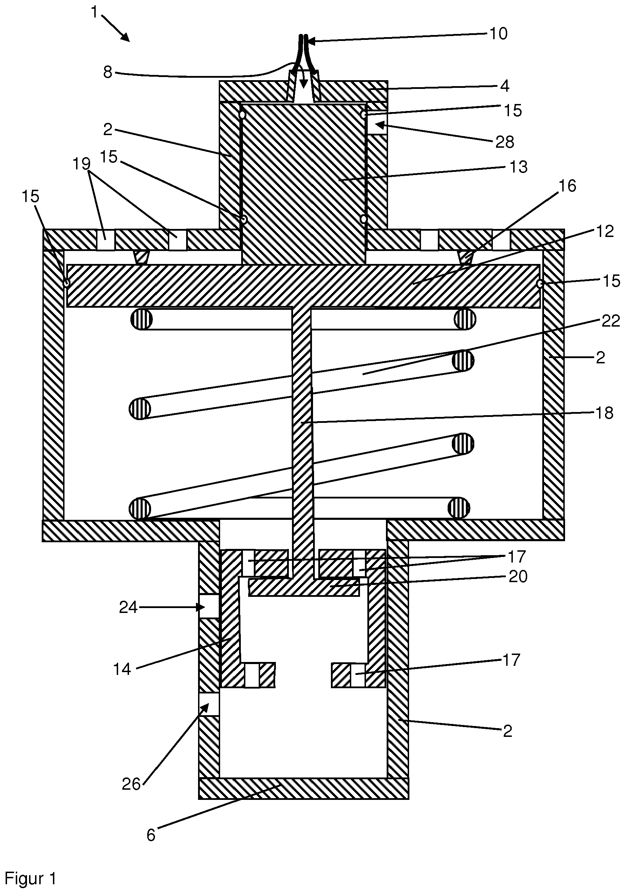

Die Figuren 1 bis 6 zeigen schematische Querschnittansichten eines erfindungsgemäßen Vakuummotors 1 während eines Arbeitszyklus in chronologischer Abfolge. Die Querschnitte enthalten die Symmetrieachse der Bauteile des Vakuummotors 1, das heißt, der Querschnitt ist mittig geschnitten dargestellt. Der Vakuummotor 1 weist einen Hohlkörper 2 aus Kunststoff mit einem dreiteiligen zylindrischen Innenraum auf. Der Innenraum ist auf der Vorderseite durch eine Deckplatte 4 und auf der Rückseite durch eine Rückplatte 6 geschlossen. In der Deckplatte 4 ist eine Ausstoßöffnung 8 zum Austreiben eines Flüssigkeitsstrahls vorgesehen. Der Vakuummotor 1 ist also zum Erzeugen eines gepulsten Strahls einer Spülflüssigkeit aus der Ausstoßöffnung 8 geeignet und vorgesehen.

Die Ausstoßöffnung 8 ist durch eine Lippenventil 10 geschlossen, das sich öffnet, wenn eine Flüssigkeit aus dem vorderen Innenraum ausgestoßen wird, das heißt, wenn der Druck im vorderen Innenraum ausreichend groß ist. Das heißt, dass der Druck im vorderen Innenraum größer ist als der Umgebungsdruck und die elastische Kraft des Lippenventils 10. Die Rückplatte 6 schließt den rückseitigen also hinteren Innenraum des Hohlkörpers 2 gasdicht und druckdicht ab. In dem mittleren Innenraum, der als Arbeitsraum bezeichnet werden kann, befindet sich ein entlang der Zylinderachse des zylindrischen Innenraums beziehungsweise des mittleren Innenraums beweglich angeordneter Arbeitskolben 12. In dem vorderen Innenraum, der als Pumpraum bezeichnet werden kann, befindet sich ein entlang der Zylinderachse des zylindrischen Innenraums beziehungsweise des vorderen Innenraums beweglich angeordneter zylindrischer Pumpkolben 13. In dem hinteren Innenraum, der als Ventilraum bezeichnet werden kann, durch den aber auch das Gas zum Leisten der Arbeit des Vakuummotors 1 geschleust wird, befindet sich ein entlang der Zylinderachse des zylindrischen Innenraums beziehungsweise des hinteren Innenraums beweglich angeordneter Steuerkolben 14. Der Arbeitskolben 12 liegt vollumfänglich an den Zylinderwänden des mittleren Innenraums an und ist dazu durch einen Dichtring 15 aus Gummi oder einem anderen elastischen Material gegen die Innenwände des mittleren Innenraums abgedichtet. Der Pumpkolben 13 liegt vollumfänglich an den Zylinderwänden des vorderen Innenraums an und ist dazu mit zwei Dichtringen 15 aus Gummi oder einem anderen elastischen Material gegen die Innenwände des vorderen Innenraums abgedichtet. Der Steuerkolben 14 liegt vollumfänglich dicht an den Zylinderwänden des hinteren Innenraums an. Auch der Steuerkolben 14 kann mit Dichtungsringen (nicht gezeigt) gegen die Innenwand des hinteren Innenraums abgedichtet sein. Die Dichtringe 15 liegen vollumfänglich an dem Arbeitskolben 12 und dem Pumpkolben und den Innenwänden des Hohlkörpers 2 an. Der Arbeitskolben 12 und der Pumpkolben 13 sind bevorzugt einteilig aufgebaut und aus Kunststoff gefertigt.

Der mittlere Innenraum ist im Durchmesser senkrecht zur Zylinderachse etwa dreimal so groß wie die Durchmesser des vorderen Innenraums und des hinteren Innenraums und damit in der Querschnittsfläche etwa neunmal so groß wie die Querschnittsfläche des vorderen Innenraums und des hinteren Innenraums. Dadurch weist der mittlere Innenraum eine vordere Vorderwand und eine hintere (rückseitige) Rückwand auf, die die Wandungen 2 des mittleren Innenraums mit den Wandungen 2 des vorderen und hinteren Innenraums verbinden. Der Arbeitskolben 12 ist von der Vorderwand des mittleren Innenraums mit mehreren Abstandhaltern 16 beabstandet, die dafür sorgen, dass der Arbeitskolben 12 nicht an der Vorderwand des mittleren Innenraums anliegen und sich dort nicht festsaugen kann. Zudem sind in der Vorderwand des mittleren Innenraums Belüftungsöffnungen 19 vorgesehen, die einen Gasaustausch zwischen der Umgebung und dem Bereich zwischen der Vorderwand des mittleren Innenraums und dem Arbeitskolben 12 ermöglichen. Dadurch behindert ein Unterdruck in diesem Bereich nicht die Bewegung des Arbeitskolbens 12 von der Vorderwand des mittleren Innenraums weg, beziehungsweise behindert ein Überdruck in diesem Bereich nicht die Bewegung des Arbeitskolbens 12 zur Vorderwand des mittleren Innenraums hin. Es kann daher vorgesehen sein, dass die Vorderwand des mittleren Innenraums durch ein Gitter realisiert ist. Dann ist der Gasaustausch besonders Luftströmungswiderstandsfrei möglich.

Der Steuerkolben 14 ist ebenfalls aus Kunststoff gefertigt und hat die Grundform eines Rohrs mit einer vorderen und einer hinteren Abdeckung, die senkrecht zur Zylinderachse ausgerichtet sind. Selbstverständlich können die Abdeckungen auch andere Formen haben und anders geneigt sein. In den Abdeckungen sind Durchgänge 17 angeordnet, die einen Gasaustausch zwischen den beiden Seiten des Steuerkolbens 14 ermöglichen sollen. Ein weiterer zentraler Durchgang auf der Symmetrieachse ist innerhalb des Abstandhalters 16 vorgesehen.

Der Steuerkolben 14 ist zwischen dem Arbeitskolben 12 und der Rückplatte 6 im hinteren Innenraum beweglich angeordnet. An dem Arbeitskolben 12 ist eine Mitnehmereinheit bestehend aus einem Mitnehmerstift 18 und einem Mitnehmer 20 befestigt. Der Mitnehmerstift 18 ist bevorzugt einteilig mit dem Arbeitskolben 12 ausgeführt und ebenfalls aus Kunststoff gefertigt. Der Mitnehmerstift 18 ist eine zylindrische Stange, die durch den axialen Durchgang in der vorderen Abdeckung des Steuerkolbens 14 reicht. Der Mitnehmer 20 ist eine flache Scheibe, die nicht durch die zentralen Durchgänge in den Abdeckungen des Steuerkolbens 14 passt. Die Form des Mitnehmers 20 ist nicht wesentlich und kann, muss aber nicht, an die Form der Abdeckungen des Steuerkolbens 14 angepasst sein.

Zwischen der Rückwand des mittleren Innenraums und dem Arbeitskolben 12 ist eine Stahlfeder 22 als Rückstellelement 22 im mittleren Innenraum angeordnet. Der Steuerkolben 14 deckt eine seitliche Gaseinlassöffnung 24 ab, die mit einem Druckgas verbunden ist oder die, erfindungsgemäß bevorzugt, zur Umgebung des Vakuummotors 1 offen ist. In letzterem Fall kann dann Umgebungsluft mit dem Luftdruck der Umgebung in den Vakuummotor 1 eingespeist werden, wenn die Gaseinlassöffnung 24 geöffnet ist. Die Gaseinlassöffnung 24 und eine Gasauslassöffnung 26 befinden sich in der Seitenwand (dem Zylindermantel) des Hohlraums 2 im hinteren Innenraum. Die Stahlfeder 22 drückt den Arbeitskolben 12 auf die Abstandhalter 16 an der Vorderwand des mittleren Innenraums, so dass der Steuerkolben 14 in der gezeigten Position gehalten wird, wenn kein Vakuum oder kein Unterdruck (ein Gas mit einem Druck unterhalb des Umgebungsdrucks) durch eine Gasauslassöffnung 26 in den hinteren und von dort aus auch den mittleren Innenraum geleitet wird. Es reicht auch aus, dass die Druckfeder 22 während des Arbeitsprozesses des Vakuummotors 1 eine eine ausreichende Rückstellkraft auf den Arbeitskolben 12 bewirkt. Bevorzugt ist die Stahlfeder 22 so lange, dass sie in dem in Figur 1 dargestellten Ausgangszustand den Arbeitskolben 12 auf die Abstandhalter 16 drückt. Dadurch kann sichergestellt werden, dass die Gasauslassöffnung 26 offen bleibt, wenn kein Unterdruck an dem Vakuummotor 1 angelegt wird und der Vakuummotor 1 dadurch immer in den Ausgangszustand nach Figur 1 überführt wird.

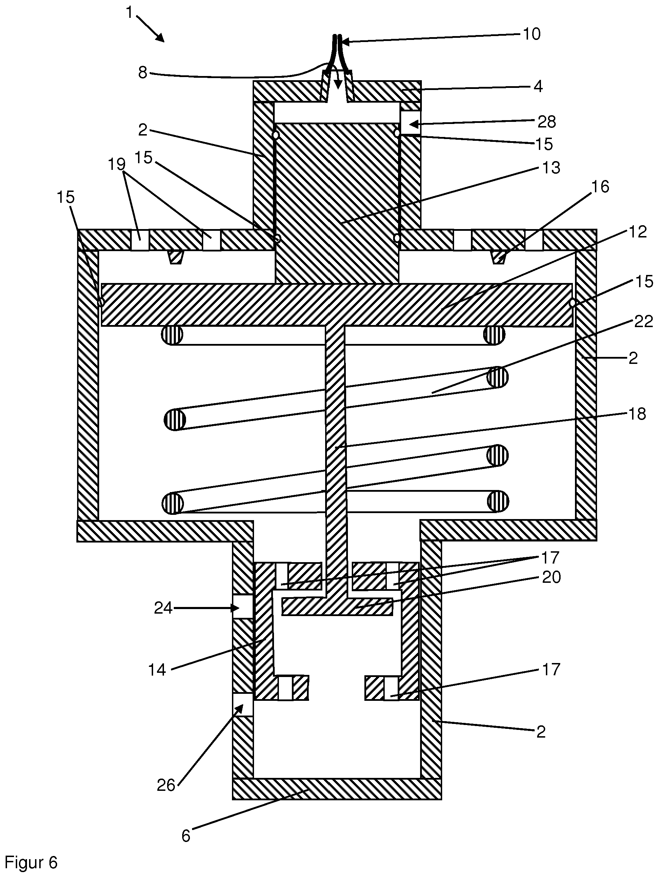

Die Gaseinlassöffnung 24 ist eine durchgehende Öffnung in der Seitenwand des Hohlkörpers 2. Neben der Gaseinlassöffnung 24 sind in der Seitenwand des Hohlkörpers 2 noch die Gasauslassöffnung 26 und eine Flüssigkeitszufuhröffnung 28 vorgesehen. Die Flüssigkeitszufuhröffnung 28 befindet sich in der Seitenwand beziehungsweise der Zylindermantelwand des vorderen Innenraums, also im Pumpraum. Die Gasauslassöffnung 26 ist in dem Ausgangszustand des Vakuummotors 1, wie er in Figur 1 gezeigt ist, durch den Steuerkolben 14 unverschlossen beziehungsweise nicht abgedeckt. Durch die Gasauslassöffnung 26 wird das Arbeitsmedium (das Gas beziehungsweise die Luft) aus dem Innenraum herausgezogen, wenn die Gasauslassöffnung 26 durch eine geeignete Position des Steuerkolbens 14 freigelegt ist (wie in Figur 1 gezeigt). Die Flüssigkeitszufuhröffnung 28 ist dagegen zwischen dem Arbeitskolben 12 beziehungsweise dem Pumpkolben 13 und der Deckplatte 4 angeordnet und ist dazu vorgesehen, den vorderen Innenraum mit einer Flüssigkeit zu füllen, die bei einer Bewegung des Arbeitskolbens 12 beziehungsweise des Pumpkolbens 13 auf die Deckplatte 4 zu (wie in Figur 6 gezeigt) durch die Ausstoßöffnung 8 ausgepresst wird und so einen Sprühstrahl beziehungsweise Spülflüssigkeitskegel (nicht gezeigt) erzeugt.

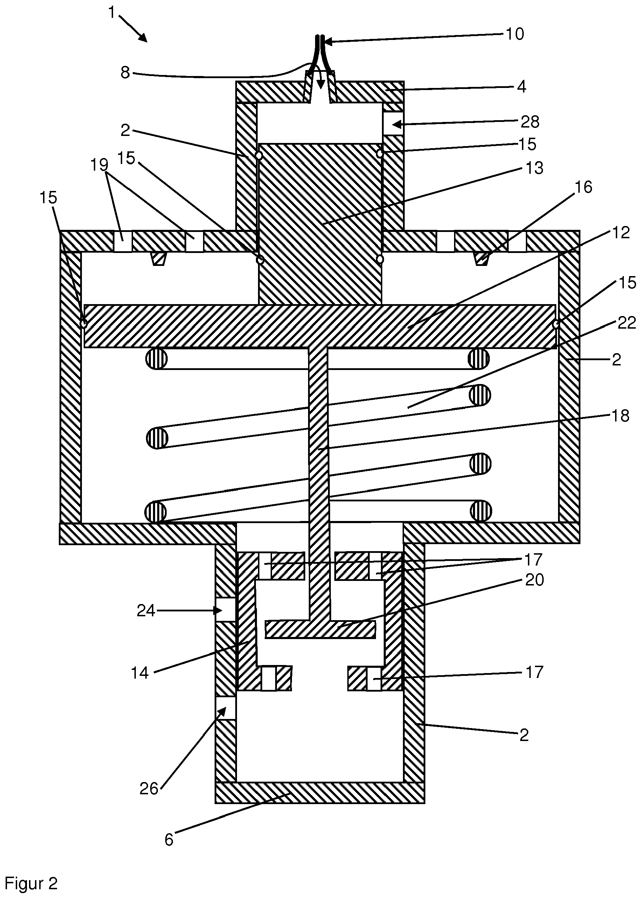

In Figur 1 ist der Ausgangszustand des Vakuummotors 1 gezeigt. Ein Unterdruck wird über die Gasauslassöffnung 26 an den Innenraum zwischen den Arbeitskolben 12 und der Rückplatte 6 angelegt. Der in diesem Zwischenraum abnehmende Druck beschleunigt den Arbeitskolben 12 in Richtung der Rückplatte 6, also der Rückseite des Vakuummotors 1. Genaugenommen beschleunigt die Druckdifferenz zwischen der Vorderseite des Arbeitskolbens 12 im mittleren Innenraum und der Rückseite des Arbeitskolbens 12 im mittleren Innenraum den Arbeitskolben 12 in Richtung der Rückplatte 6. Der Steuerkolben 14 bleibt dabei an Ort und Stelle. Bei der Bewegung des Arbeitskolbens 12 wird die Druckfeder 22 zusammengedrückt und gespannt. Die Druckfeder 22 nimmt dabei Energie auf. Diese Situation ist in Figur 2 dargestellt.

Durch die Bewegung des Pumpkolbens 13 wird eine Flüssigkeit durch die Flüssigkeitszufuhröffnung 28 in den vorderen Innenraum gezogen. Dazu ist der vordere Innenraum durch den Pumpkolben 13 mit den Dichtungen 15 und den Hohlraum 2 mit der Deckplatte 4 bis auf die Flüssigkeitszufuhröffnung 28 geschlossen. Das Lippenventil 10 schließt wegen des Unterdrucks im vorderen Innenraum. Die Flüssigkeitszufuhr wird so lange stattfinden, wie sich der Arbeitskolben 12 und damit der Pumpkolben 13 in Richtung der Rückplatte 6 bewegen.

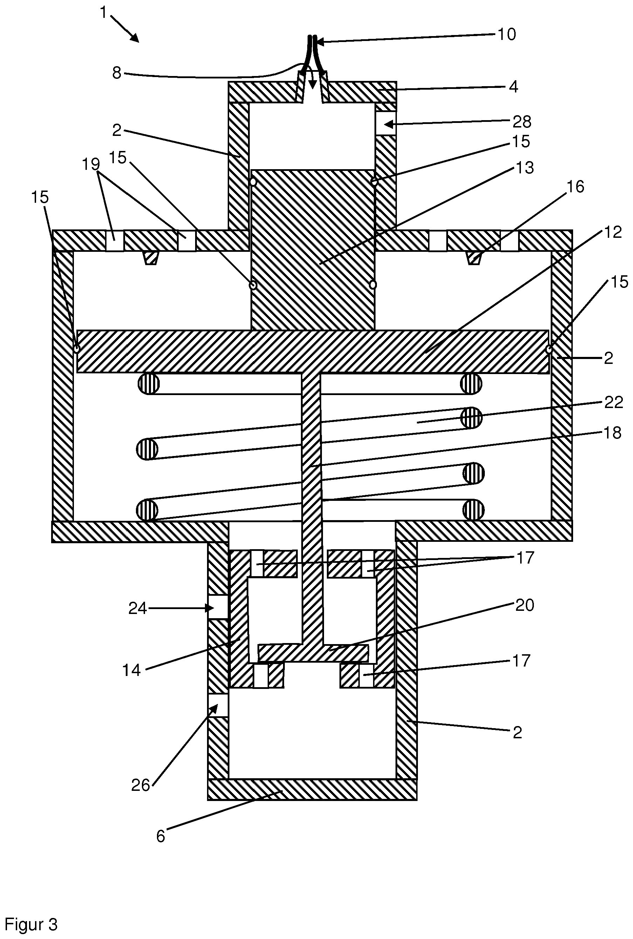

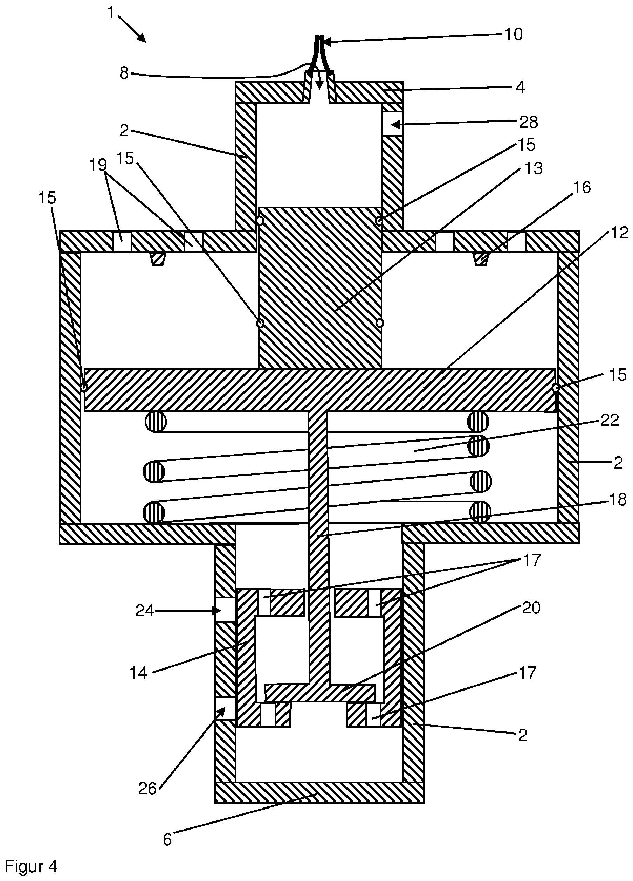

Während der Bewegung des Arbeitskolbens 12 drückt dieser das Federelement 22 zusammen. Wenn sich der Arbeitskolben 12 so weit zum Steuerkolben 14 hin bewegt hat, dass die Länge bis zum hinteren Anschlag an der rückseitigen Abdeckung durch den Mitnehmerstift 18 und den Mitnehmer 20 überbrückt ist, trifft der Mitnehmer 20 auf die rückseitige Abdeckung des Steuerkolbens 14 (diese Situation ist in Figur 3 dargestellt) und schlägt diesen nach hinten. Der Steuerkolben 14 wird dann von dem Arbeitskolben 12 mit Hilfe des Mitnehmerelements 18, 20 mitgeschoben. Dadurch bewegt sich der Steuerkolben 14 zur Rückplatte 6 hin. Durch die Bewegung des Steuerkolbens 14 wird die Gasauslassöffnung 26 abgedeckt beziehungsweise geschlossen. Diese Situation ist in Figur 4 dargestellt.

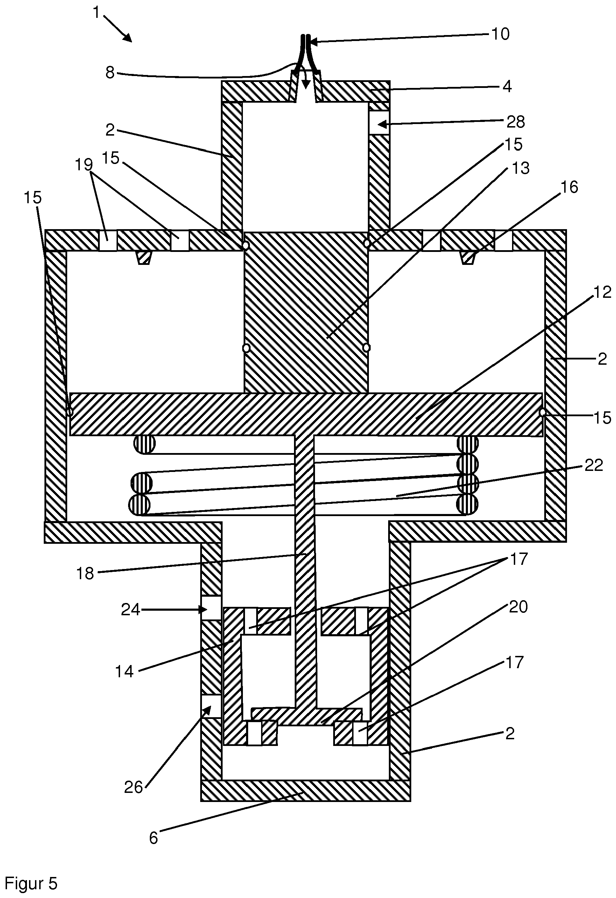

Während der ganzen Zeit wird der vordere Innenraum zwischen dem Pumpkolben 13 und der Deckplatte 4 durch die Flüssigkeitszufuhröffnung 28 mit Spülflüssigkeit gefüllt. Durch die Trägheit des Arbeitskolbens 12 beziehungsweise des Steuerkolbens 14 und/oder dem immer noch zwischen dem Arbeitskolben 12 und der Rückplatte 6 existierenden Unterdruck im Innenraum wird der Steuerkolben 14 noch weiter nach hinten bewegt und die Gaseinlassöffnung 24 geöffnet. Diese Situation ist in Figur 5 dargestellt.

Anschließend strömt das Gas aus der Umgebung oder aus einem Druckgasreservoir durch die Gaseinlassöffnung 24 In den Innenraum zwischen dem Arbeitskolben 12 und der Rückplatte 6. Das Federelement 22 beschleunigt den Arbeitskolben 12 in Richtung der Deckplatte 4. Durch das sich verringernde Volumen zwischen dem Pumpkolben 13 und der Deckplatte 4 entsteht dort ein Überdruck und das Lippenventil 10 öffnet. Bevorzugt ist an der Flüssigkeitszufuhröffnung 28 ein Rückschlagventil (nicht gezeigt) angeordnet, das ein Austreten der Flüssigkeit aus dem Zwischenraum zwischen dem Arbeitskolben 12 und der Deckplatte 4 in die Flüssigkeitszufuhr verhindert. Die Flüssigkeit oder das Flüssigkeits-Gas-Gemisch in dem vorderen Innenraum wird mit dem Pumpkolben 13 aus dem vorderen Innenraum durch die Ausstoßöffnung 8 herausgedrückt beziehungsweise herausgepresst. Ein Sprühstrahl der Spülflüssigkeit entsteht.

Der Arbeitskolben 12 löst durch seine Bewegung den Mitnehmer 20 von dem Anschlag der hinteren Abdeckung des Steuerkolbens 14. Der Arbeitskolben 12 trifft schließlich auf die vordere Abdeckung des Steuerkolbens 14. Dadurch wird auch der Steuerkolben 14 wieder in Richtung der Deckplatte 4 bewegt und die Gaseinlassöffnung 24 schließt sich wieder und die Gasauslassöffnung 26 beginnt sich zu öffnen. Dieser Zustand ist in Figur 6 gezeigt.

Nach einer vollständigen Öffnung der Gasauslassöffnung 26 hat das Federelement 22 den Arbeitskolben 12 und den Steuerkolben 14 wieder in den in Figur 1 gezeigten Ausgangszustand überführt. Währenddessen ist die Spülflüssigkeit oder das Flüssigkeit-Gas-Gemisch aus dem Zwischenraum zwischen dem Arbeitskolben 12 und der Deckplatte 4 vollständig durch die Ausstoßöffnung 8 ausgestoßen worden. Der Zyklus beginnt von vorn.

Der Steuerkolben 14 und dessen Bewegung bewirken eine automatische Ventilsteuerung der Gaseinlassöffnung 24 und der Gasauslassöffnung 26, so dass der Steuerkolben 14 als Ventilelement aufgefasst werden kann.

Durch Einwirkung des Rückstellelements 22 wird bei der Bewegung des Arbeitskolbens 12 in Richtung der Deckplatte 4 die im Hohlraum vorhandene Flüssigkeit durch die Ausstoßöffnung 8 und das Auslassventil 10 heraus gepresst. Ein Rückschlagventil (nicht gezeigt), das an der Flüssigkeitszufuhröffnung 28 angeordnet ist, verhindert ein Austritt der Flüssigkeit in das Flüssigkeitsreservoir. Nach Öffnung der Gasauslassöffnung 26 und der Bewegung des Arbeitskolbens 12 in Richtung der Rückplatte 6 wird durch Einwirkung des Vakuums beziehungsweise des Unterdrucks in Verbindung mit dem Druckgas oder dem Umgebungsdruck ein Unterdruck im vorderen Innenraum erzeugt. Dabei öffnet sich das Rückschlagventil und die Flüssigkeit fließt in den vorderen Innenraum.

Nach Schließen der Gasauslassöffnung 26 drückt das Rückstellelement 22 den Arbeitskolben 12 zurück in seine Ausgangslage, wobei durch den Überdruck das Rückschlagventil schließt und Flüssigkeit aus dem vorderen Innenraum gedrückt wird.

Danach läuft der Pumpprozess der Flüssigkeit in der beschriebenen Weise solange ab, wie ein Vakuum oder ein Unterdruck an der Gasauslassöffnung 26 anliegt und sich Flüssigkeit im Flüssigkeitsreservoir befindet.

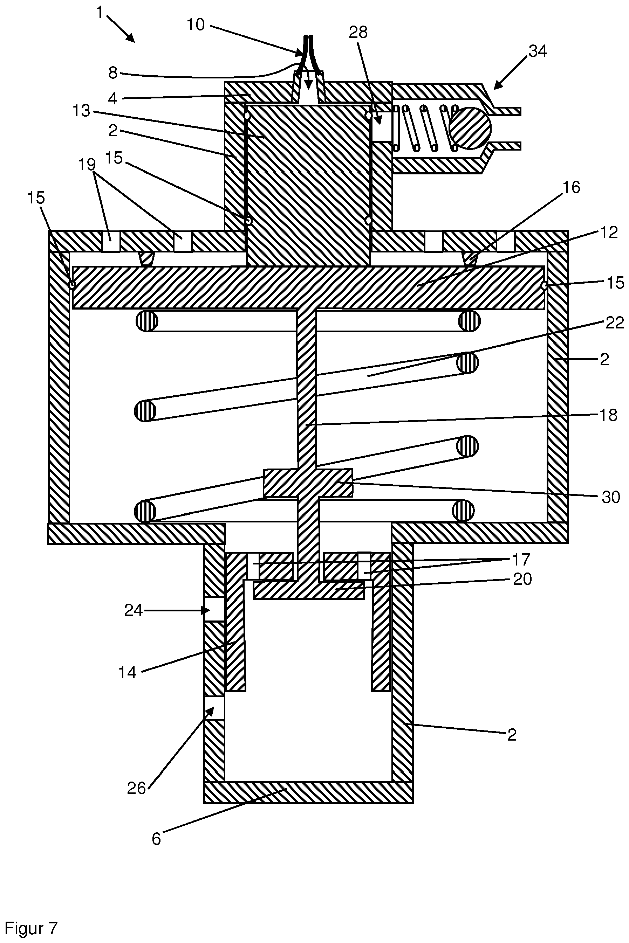

Figur 7 zeigt eine schematische Querschnittansicht eines alternativen erfindungsgemäßen Vakuummotors 1 mit einem Abstandhalter 30 am Mitnehmerstift 18. Der Abstandhalter 30 kann sich als Strebe in beide Richtungen radial vom Mitnehmerstift 18 weg erstrecken oder durch eine Kreisscheibe gebildet sein, die sich radial senkrecht vom Mitnehmerstift 18 ins Innere des mittleren Innenraums oder des hinteren Innenraums (je nach Position des Arbeitskolbens 12 im Hohlkörper 2) erstreckt. Der Steuerkolben 14 benötigt bei dieser Ausführung nur eine vordere Abdeckung. Im Vergleich zu der Ausführungsform nach den Figuren 1 bis 6 wird bei dieser Ausführungsform nach Figur 7 also auf eine hintere Abdeckung am Steuerkolben 14 verzichtet. Dennoch hat der Steuerkolben 14 dieses Vakuummotors 1 zwei Anschläge für den Mitnehmer 20 und den Abstandhalter 30 an dem Mitnehmerstift 18, die bei dieser Ausführung durch die Vorderseite und die Rückseite der einzigen (vorderen) Abdeckung gebildet sind. Bei der Ausführung nach den Figuren 1 bis 6 werden diese beiden Anschläge stattdessen durch die Rückseite der vorderen Abdeckung und durch die Vorderseite der hinteren Abdeckung gebildet.

Die Durchführung durch den Steuerkolben 14 des Vakuummotors 1 nach Figur 7, durch die sich der Mitnehmerstift 18 erstreckt, dient auch dem Gasaustausch des Zwischenraums zwischen dem Arbeitskolben 12 und dem Steuerkolben 14 und dem Zwischenraum zwischen dem Steuerkolben 14 und der Rückplatte 6. Auf zusätzliche Durchgänge 17 könnte theoretisch verzichtet werden. Um Arbeit am Arbeitskolben 12 zu leisten, also um das Vakuum auf der Rückseite des Arbeitskolbens 12 wirken zu lassen, ist es jedoch wichtig, dass eine ausreichend gasdurchlässige Verbindung vorhanden ist, die einen ausreichenden Querschnitt beziehungsweise einen geringen Gasstromwiderstand aufweist. Hierzu können neben den Durchgängen 17 und der Durchführung für den Mitnehmerstift 18 auch die Zwischenräume zwischen dem Steuerkolben 14 und der inneren Zylinderwand des hinteren Innenraums verwendet werden. Zu diesem Zweck können Ausnehmungen vorgesehen sein, die sich entlang der gesamten Länge der Außenwände des Steuerkolbens 14 erstrecken und/oder die sich entlang der gesamten Länge des Hubraums des Steuerkolbens 14 in den Innenwänden 2 des hinteren Innenraums erstrecken.

Zudem ist bei dem Vakuummotor 1 in Figur 7 auch ein Rückschlagventil 34 an der Flüssigkeitszufuhröffnung 28 eingezeichnet. Ansonsten gleichen der Aufbau und die Funktionsweise denen, die in den Figuren 1 bis 6 dargestellt und vorstehend beschrieben sind.

Die Druckfeder 22 drückt den Arbeitskolben 12 so weit in Richtung der Deckplatte 4, dass die Vorderseite des Mitnehmers 20 an der Rückseite der Abdeckung des Steuerkolbens 14 anliegt und dadurch der Steuerkolben 14 derart in Richtung der Vorderseite (der Deckplatte 4) gezogen wird, dass die Gasauslassöffnung 26 frei liegt und die Gaseinlassöffnung 24 verschlossen ist. Bevorzugt übt die Druckfeder 22 auch dann noch eine Kraft auf den Arbeitskolben 12 aus, die den Arbeitskolben 12 in Richtung der Deckplatte 4 drückt. Beim Einwirken eines Vakuums oder eines Unterdrucks durch die Gasauslassöffnung 26 wird der Arbeitskolben 12 in Richtung des Steuerkolbens 14 beziehungsweise in Richtung der Rückplatte 6 gezogen und dabei die Druckfeder 22 gespannt. Der Steuerkolben 14 wird in Richtung der Rückplatte 6 beschleunigt, wenn der Abstandhalter 30 auf die Vorderseite der Abdeckung des Steuerkolbens 14 trifft. Der Unterscheid zu der Ausführungsform nach den Figuren 1 bis 6 besteht also darin, dass nicht die Rückseite des Mitnehmers 20 den Steuerkolben 14 beschleunigt, sondern die Rückseite des Abstandhalters 30. Die Arbeitsweise des Vakuummotors 1 nach Figur 7 entspricht ansonsten der Funktionsweise des Vakuummotors 1 nach den Figuren 1 bis 6.

Das Rückschlagventil 34, das in der gleichen Form auch an den Flüssigkeitszufuhröffnungen 28 anderer erfindungsgemäßer Vakuummotoren, wie dem nach den Figuren 1 bis 6 angeordnet sein kann, besteht aus einer Kugel, die mit einer Stahlfeder auf einen Kugelsitz gepresst wird. Dadurch wird eine Strömung in den vorderen Innenraum des Hohlkörpers 2 hinein durch die Flüssigkeitszufuhröffnung 28 ermöglicht, während eine Strömung aus dem Innenraum heraus durch das Rückschlagventil 34 blockiert wird. Wenn also der Inhalt des Vakuummotors 1 zwischen dem Pumpkolben 13 und der Deckplatte 4 durch eine Bewegung des Arbeitskolbens 12 auf die Deckplatte 4 zu durch die Ausstoßöffnung 8 ausgesprüht wird, kann der Inhalt wegen des Rückschlagventils 34 nicht in die Zuleitung vordringen.

Die druckgasgetriebene Flüssigkeitspumpe 1 funktioniert in der Weise, dass auf der Stirnseite des Pumpkolbens 13 ein Hohlraum (als Teil des vorderen Innenraums) vorhanden ist. Dieser Hohlraum ist mit dem Rückschlagventil 34 flüssigkeitsdurchlässig verbunden, das einen Flüssigkeitseinstrom aus einem Flüssigkeitsreservoir in den Hohlraum ermöglicht und ein Zurückströmen der Flüssigkeit aus dem Hohlraum in Richtung Flüssigkeitsreservoir verhindert. Weiterhin ist ein Auslassventil 10 flüssigkeitsdurchlässig mit dem Hohlraum verbunden. Dieses ermöglicht ein Ausströmen der Flüssigkeit aus dem Hohlraum und verhindert ein Zurückströmen der Flüssigkeit zurück in den Hohlraum. Dieses Auslassventil 10 kann im einfachsten Fall ein Lippenventil 10 sein. Die grundsätzliche Funktionsweise des Vakuummotors 1 erfolgt in der beschriebenen Weise.

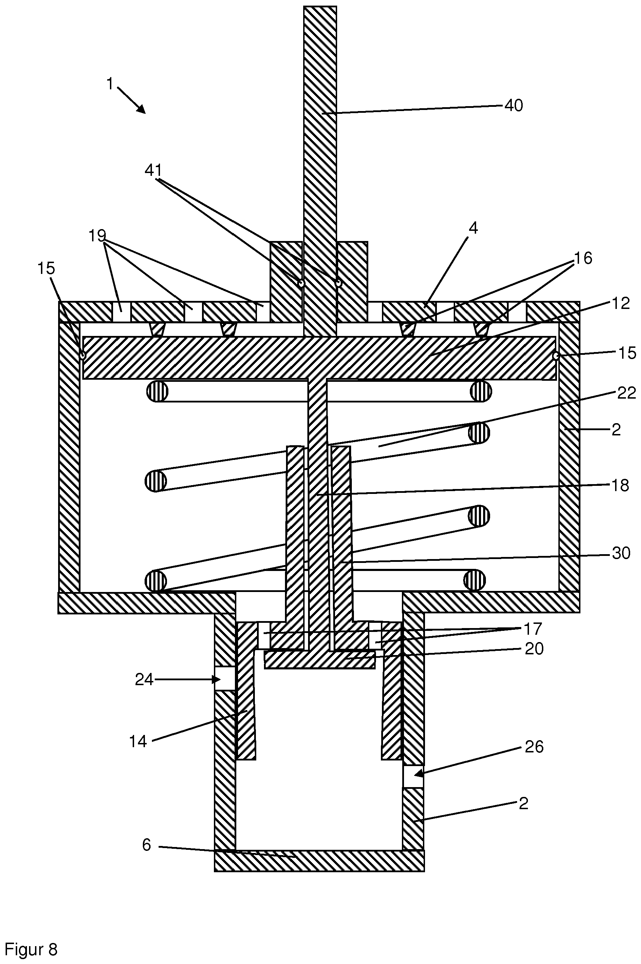

Figur 8 zeigt eine schematische Querschnittansicht eines dritten alternativen erfindungsgemäßen Vakuummotors 1 mit einem Abstandhalter 30, der an einem Steuerkolben 14 angeordnet ist, beziehungsweise der einteilig mit dem Steuerkolben 14 ausgeführt ist, und mit einer Stange 40 zur Bildung eines stoßenden Vakuummotors 1. Der Aufbau des Vakuummotors 1 gleicht dem der zuvor beschriebenen Vakuummotoren nach den Figuren 1 bis 6 und 7, wobei zusätzlich zu den genannten Unterschieden die folgenden Unterschiede vorhanden sind. Der Vakuummotor 1 weist einen Hohlkörper 2 mit einem nur zweiteiligen zylindrischen Innenraum auf. Der vordere Innenraum ist auf der Vorderseite mit einer Deckplatte 4 abgedeckt und der hintere Innenraum ist auf der Rückseite mit einer Rückplatte 6 gas- und druckdicht abgeschlossen. Im Vergleich zu den Ausführungen nach den Figuren 1 bis 7 fehlt also der vorderste Innenraum (der mittlere Innenraum nach den Figuren 1 bis 7 entspricht dem vorderen Innenraum nach Figur 8).

Im vorderen Innenraum ist ein Arbeitskolben 12 linear beweglich entlang der Zylinderachse des Innenraums angeordnet. Zwischen dem Arbeitskolben 12 und der Rückplatte 6 ist der Steuerkolben 14 linear beweglich entlang der Zylinderachse des hinteren Innenraums angeordnet. Arbeitskolben 12 und Steuerkolben 14 weisen einen zylindrischen Außenmantel auf, der dem inneren Zylindermantel des Innenraums entspricht.

Der Arbeitskolben 12 ist mit einem Dichtungsring 15 gegen die Innenwand des vorderen Innenraums abgedichtet. Der vordere Innenraum weist einen etwa neunmal so großen Querschnitt auf als der hintere Innenraum. Dementsprechend hat der Arbeitskolben 12 einen etwa dreimal größeren Durchmesser als der Steuerkolben 14. An der Rückseite der Deckplatte 4 sind mehrere kurze Abstandhalter 16 in Form von Kegelstümpfen angeordnet, die ein Anliegen des Arbeitskolbens 12 an der Deckplatte 4 verhindern sollen. Zudem weist die Deckplatte 4 Belüftungsöffnungen 19 auf, durch die Luft aus der Umgebung des Vakuummotors 1 eingezogen werden kann. Dadurch wird erreicht, dass in dem Zwischenraum zwischen der Vorderseite des Arbeitskolbens 12 und der Deckplatte 4 keine oder nur sehr geringe Druckschwankungen entstehen, die einer Bewegung des Arbeitskolbens 12 entgegengerichtet wären. In dem Steuerkolben 14 sind Durchgänge 17 angeordnet, die einen Gasaustausch zwischen der Vorderseite und der Rückseite des Steuerkolbens 14 ermöglichen.

An dem Arbeitskolben 12 ist auf der Rückseite ein Mitnehmerelement angeordnet, das aus einem Mitnehmerstift 18 und einem Mitnehmer 20 besteht. Der Mitnehmerstift 18 reicht durch eine Durchführung im Steuerkolben 14, so dass der Mitnehmer 20 auf der Rückseite einer Fläche senkrecht zur Zylinderachse des Steuerkolbens 14 eingreifen kann, um den Steuerkolben 14 beabstandet zum Arbeitskolben 12 mitzuziehen, wenn sich der Arbeitskolben 12 in Richtung der Vorderseite, also der Deckplatte 4 bewegt. Diese Fläche beziehungsweise dieser Anschlag entspricht der Rückseite der vorderen Abdeckung nach den Figuren 1 bis 6 und der einzigen Abdeckung nach Figur 7.

Anstatt einen Abstandhalter am Mitnehmerstift 18 vorzusehen (wie in dem Ausführungsbeispiel nach Figur 7 vorgeschlagen) ist bei der Ausführung nach Figur 8 ein Abstandhalter 30 in Form einer zylindrischen Rohrs am Steuerkolben 14 vorgesehen, dessen vordere Fläche als zweiter Anschlag dient, auf den der Arbeitskolben 12 bei einer Bewegung in Richtung der Rückplatte 6 trifft. Der Abstandhalter 30 kann auch durch Stangen oder eine andere geometrische Form gebildet werden, die den Gasaustausch zwischen der Vorderseite und der Rückseite des Steuerkolbens 14 und die Bewegung des Federelements 22 und des Arbeitskolbens 12 nicht behindert.

In dem vorderen Innenraum zwischen dem Arbeitskolben 12 und der Rückplatte 6 beziehungsweise der Rückwand des vorderen Innenraums ist ein Federelement 22 als Druckfeder 22 um den Mitnehmerstift 18 herum angeordnet, die den Arbeitskolben 12 in Richtung der Deckplatte 4 drückt. In Figur 8 ist also die Ausgangsstellung des Vakuummotors 1 gezeigt.

In der Seitenwand des Hohlkörpers 2 im hinteren Innenraum sind zwei durchgehende Öffnungen 24, 26 vorgesehen. Die Gaseinlassöffnung 24 ist nach außen offen, so dass Umgebungsluft in den Vakuummotor 1 eingespeist werden kann. Während des Arbeitszyklus des Vakuummotors 1 wird die Luft aus dem Innenraum des Hohlkörpers 2 hinter dem Arbeitskolben 12 durch die Gasauslassöffnung 26 ausgesaugt beziehungsweise teilweise oder weitgehend evakuiert. Der Arbeitszyklus läuft ab, wie mit dem ersten Ausführungsbeispiel nach den Figuren 1 bis 6 beschrieben.

Anstatt eine Flüssigkeit periodisch auszustoßen, wie mit den Lavage-Pumpen nach den Figuren 1 bis 7, wird mit dem Vakuummotor 1 nach Figur 8 ein periodisches Stoßen der Stange 40 erreicht. Die Stange 40 ist mit einer Führungshülse 41 durch eine Öffnung in der Deckplatte 4 aus dem Innenraum heraus durchgeführt. Alternativ kann anstatt der starr mit dem Arbeitskolben 12 verbundenen Stange 40 kann auch eine Pleuelstange 40 über ein Gelenk (nicht gezeigt) mit dem Arbeitskolben 12 verbunden sein, die durch die Deckplatte 4 durchgeführt ist. Mit der Pleuelstange 40 kann die lineare periodische Bewegung mit Hilfe einer Kurbelwelle (nicht gezeigt), die vorne an der Pleuelstange 40 über ein Gelenk befestigt ist, in eine Drehbewegung übersetzt werden. Dementsprechend weist der Vakuummotor 1 nach Figur 8 keinen Pumpkolben auf.

Der Vakuummotor 1 nach Figur 8 kann ebenfalls in einem Lavage-System eingesetzt werden. Dazu wird die Spitze der Stange 40 dazu eingesetzt, auf eine Membran (nicht gezeigt) zu stoßen. Die periodischen Druckstöße auf die Membran können zum periodischen Ausstoß einer Flüssigkeit verwendet werden. Ebenso kann die periodische Bewegung der Stange 40 als Antrieb für einen Rüttelmechanismus oder einen Klopfermotor verwendet werden.

Alternativ kann der Vakuummotor 1, wenn er mit einer Pleuelstange 40 ausgeführt ist, zum Antrieb einer Trennscheibe oder einer Pumpe verwendet werden.

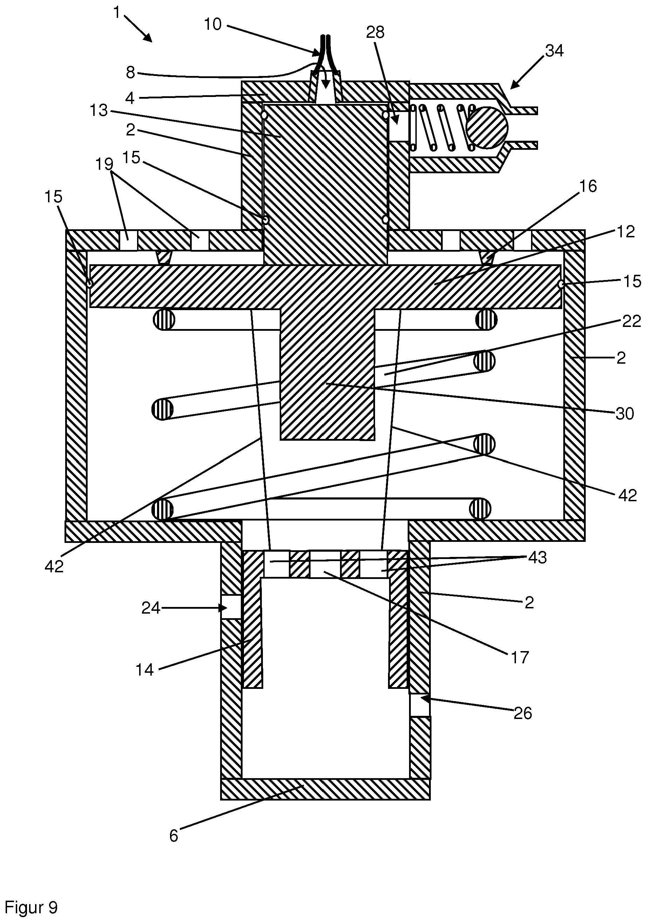

Figur 9 zeigt eine schematische Querschnittansicht eines vierten alternativen erfindungsgemäßen Vakuummotors 1 mit einer Schnur 42 oder einem Kabel 42 als Mitnehmerelement 42 und mit einem Rückschlagventil 34. Der Aufbau und die Anordnung des Rückschlagventils 34 entsprechen den nach Figur 7 beschriebenen. Bei dieser Version des Vakuummotors 1 ist ein Abstandhalter 30 als massiver Zylinder auf der Rückseite des Arbeitskolbens 12 angeordnet. Der Arbeitskolben 12 ist wieder über einen O-Ring 15 gegen die Innenwände des mittleren zylindrischen Innenraums im Hohlkörper 2 abgedichtet.

Bei der in Figur 9 gezeigten Stellung des Vakuummotors 1 sind die Kabel 42 gespannt. Der Arbeitskolben 12 zieht den Steuerkolben 14 mit den Kabeln 42 hinter sich her in Richtung der Deckplatte 4, um die Gaseinlassöffnung 24 zu schließen und die Gasauslassöffnung 26 danach zu öffnen. Die Kabel 42 sind an Streben 43 an der Vorderseite des Steuerkolbens 14 befestigt, beispielsweise mit einer Schlaufe um die Streben 43 herum gebunden.

Wenn der Arbeitskolben 12 durch das Vakuum oder den Unterdruck in Richtung der Rückplatte 6 gezogen wird, hängen die Kabel 42 lose im mittleren Innenraum zwischen dem Arbeitskolben 12 und dem Steuerkolben 14 herum. Nur beim Nachziehen des Steuerkolbens 14 durch den Arbeitskolben 12 sind die Kabel 42 oder Schnüre 42, wie in Figur 9 gezeigt, straff gespannt.

Die Vorderseite des Steuerkolbens 14 bilden dann den einzigen Anschlag, auf den der Arbeitskolben 12 beziehungsweise der Abstandhalter 30 des Arbeitskolbens 12 trifft, um den Steuerkolben 14 in Richtung der Rückplatte 6 zu stoßen und dadurch die Gasauslassöffnung 26 zu verschließen und anschließend die Gaseinlassöffnung 24 zu öffnen. Die Ausführungsform nach Figur 9 kommt also mit nur einem Anschlag aus. Die Gaseinlassöffnung 24 und die Gasauslassöffnung 26 müssen nicht nebeneinanderliegen, sondern können, wie in Figur 9 gezeigt, auch gegenüberliegend oder an anderen Stellen der passenden Höhe der Zylinderachse des hinteren Innenraums im Zylindermantel angeordnet sein.

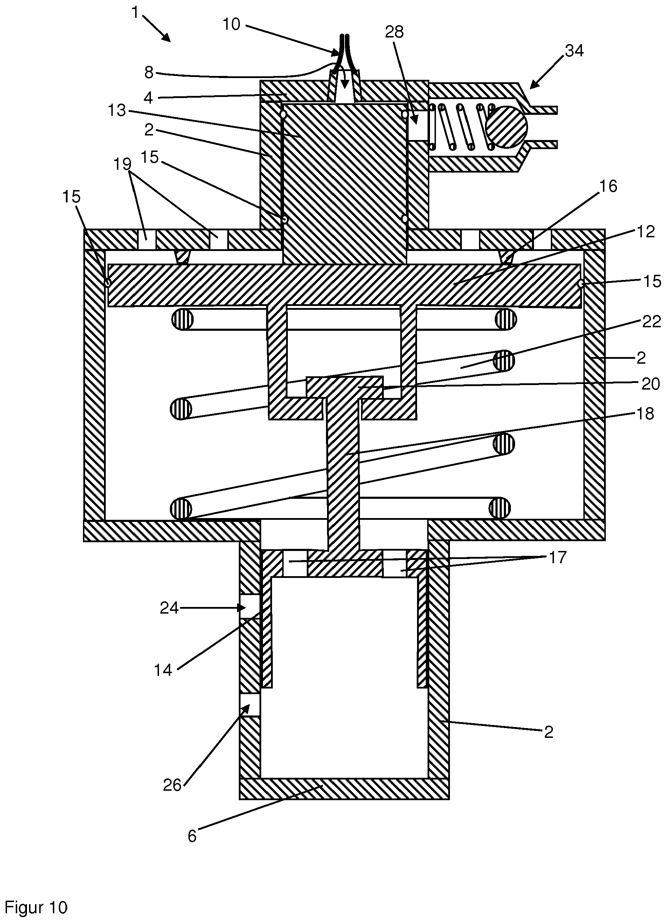

Figur 10 zeigt eine schematische Querschnittansicht eines fünften alternativen erfindungsgemäßen Vakuummotors 1, bei dem das Mitnehmerelement 18, 20 am Steuerkolben 14 befestigt ist. Das Mitnehmerelement 18, 20 läuft bei dieser Ausführungsform in einen Hohlraum im Arbeitskolben 12. Dazu ist ein Aufbau an der Rückseite des Arbeitskolbens 12 angeordnet. Da der Mitnehmerstift 18 länger ist als die Tiefe des Hohlraums im Arbeitskolben 12, übernimmt er auch die Aufgabe eines Abstandhalters mit dem sichergestellt wird, dass der Arbeitskolben 12 vom Steuerkolben 14 in jedem Fall beabstandet ist, wenn der Arbeitskolben 12 den Steuerkolben 14 in Richtung der Rückplatte 6 drückt.

Die Durchgänge 17 im Steuerkolben 14 sind notwendig, damit das Gas im Vakuummotor 1 von der Rückseite des Arbeitskolbens 12 durch den Steuerkolben 14 zur Gasauslassöffnung 26 strömen kann, um schließlich durch die Gasauslassöffnung 26 auszuströmen, wenn diese durch den Steuerkolben 14 geöffnet ist.

Die beiden Anschläge der Ausführungsform nach Figur 10 werden also bezogen auf den Steuerkolben 14 durch die Vorderseite und die Rückseite des Mitnehmers 20 gebildet und bezogen auf den Arbeitskolben 12 durch die vorderseitige Innenfläche im Hohlraum beziehungsweise in dem Aufbau auf der Rückseite des Arbeitskolbens 12 und durch die rückseitige Fläche im Hohlraum des Aufbaus auf der Rückseite des Arbeitskolbens 12 gebildet.

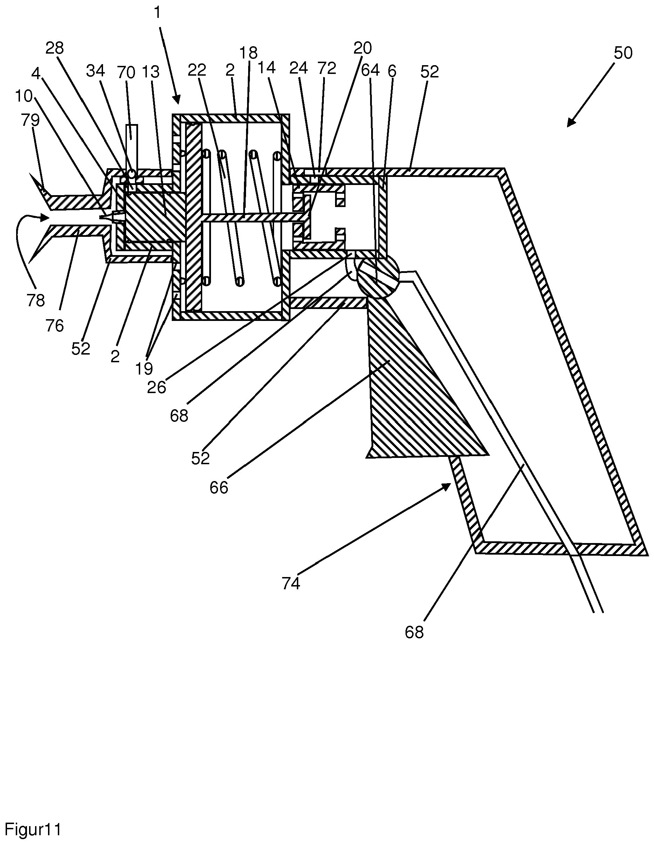

Figur 11 zeigt eine schematische Querschnittansicht eines erfindungsgemäßen in einer Hand haltbaren Lavage-Systems 50 mit einem erfindungsgemäßen Vakuummotor 1. Der Vakuummotor 1 ist bis auf die Anordnung des Gaseinlassöffnung 24 und der Gasauslassöffnung 26 relativ zueinander wie der Vakuummotor 1 nach den Figuren 1 bis 6 aufgebaut, so dass für diesen Aufbau auf dieses Ausführungsbeispiel verwiesen werden kann. Die anderen Ausführungsbeispiele bis auf den Vakuummotor nach Figur 8, können durch leichte Anpassung ebenfalls als Vakuummotor 1 oder Pumpmotor 1 in dem vorliegenden Lavage-System 50 eingesetzt werden.

Das Lavage-System 50 weist ein Gehäuse 52 aus Kunststoff auf, in dem der Vakuummotor 1 angeordnet ist. Ein Vakuum oder ein Unterdruck wird über eine Gasableitung 68 zu einem Steuerungsventil 64 geleitet. Das Steuerungsventil 64 kann mit einem Abzug 66 nach Art einer Pistole manuell mit der gleichen Hand bedient werden, mit dem das Lavage-System 50 gehalten wird. Die Gaszuleitung 68 setzt sich hinter dem manuell bedienbaren Steuerungsventil 64 fort und ist an die Gasauslassöffnung 26 des Vakuummotors 1 angeschlossen.

Die Flüssigkeitszufuhröffnung 28 ist an eine Spülflüssigkeitszuleitung 70 angeschlossen in der ein Rückschlagventil 34 angeordnet ist. Auf der Oberseite des Gehäuses 52 ist eine Öffnung 72 vorgesehen, durch die die umgebende Luft in den Vakuummotor 1 durch die Gaseinlassöffnung 24 einströmen kann. Die Öffnung 72 kann mit einem Gitter und/oder einem Filter (nicht gezeigt) abgedeckt sein, um eine Verunreinigung des Inneren des Vakuummotors 1 zu verhindern.

Die Gasableitung 68 beziehungsweise der Anschluss 68 an die Unterdruckquelle oder Vakuumquelle ist in einem Griffstück 74 nach Art eines Pistolengriffs hindurchgeführt, wobei das Griffstück 74 durch das Kunststoffgehäuse 52 gebildet wird. Ebenso kann die Flüssigkeitsleitung 70 bodenseitig aus dem Griffstück 74 des Lavage-Systems 50 herausgeführt werden (nicht gezeigt). An der Vorderseite des Lavage-Systems 50 ist ein Rohr 76 mit einer Austragsöffnung 78 und einem Trichter 79 angeordnet, durch die die Spülflüssigkeitsstöße des Vakuummotors 1 aus dem Lavage-System 50 austreten beziehungsweise abgegeben werden.

Bevorzugt ist bei solchen Lavage-Systemen 50 eine Absaugeinrichtung (nicht gezeigt) vorgesehen, mit der überschüssige Flüssigkeit und mit der Flüssigkeit abgetragene Teile über den Trichter 79 und das Rohr 76 abgesaugt und abgeleitet werden. Dazu ist vorzugsweise auch eine an der Absaugeinrichtung angeschlossene Absaugleitung bodenseitig durch das Griffstück 74 durchgeführt. Wenn Steuerungsventil 64 mit dem Abzug 66 bedient wird, wird die Gasableitung 68 durchgehend und Luft wird aus dem Vakuummotor 1 beziehungsweise dem hinteren Innenraum und dem mittleren Innenraum des Vakuummotors 1 herausgezogen. Die Druckfeder 22 beziehungsweise das Rückstellelement 22 befindet sich im mittleren Innenraum des Hohlkörpers 2. Durch die so entstehende Druckdifferenz zwischen der Vorderseite (in Figur 11 links) des Arbeitskolbens 12 und der Rückseite (in Figur 11 rechts) des Arbeitskolbens 12 werden der Arbeitskolben 12 und anschließend um eine Zeitspanne versetzt der Steuerkolben 14 in Bewegung gesetzt und der Vakuummotor 1 arbeitet, wie zuvor beschrieben. Gleichzeitig strömt die Spülflüssigkeit, die durch die Flüssigkeitszuleitung 70 aus einem externen Flüssigkeitsresevoir (nicht gezeigt) gefördert wird, periodisch in den Zwischenraum zwischen dem Pumpkolben 13 und der Deckplatte 4 des Vakuummotors 1.

Die Spülflüssigkeit wird aus dem Vakuummotor 1 mit periodischen Stößen durch das Lippenventil 10 und das Rohr 76 und die Austragsöffnung 78 ausgestoßen, solange das Vakuum oder der Unterdruck an der Gasauslassöffnung 26 anliegt und die umgebende Luft durch die Gaseinlassöffnung 24 in den Vakuummotor 1 hinein und durch diesen hindurch strömt. Der Prozess wird beendet, wenn das Steuerungsventil 64 nicht mehr bedient wird und dadurch die Gasableitung 68 unterbrochen beziehungsweise geschlossen wird. Die Rückstellung des Steuerungsventils 64 erfolgt beispielsweise mit Hilfe einer elastischen Feder (nicht gezeigt). Durch das Federelement 22 im Vakuummotor 1 werden der Arbeitskolben 12 und der Steuerkolben 14 dann in die gezeigte Ausgangsstellung (siehe auch Figur 1) gebracht und das Lavage-System 50 ist sofort wieder einsatzbereit.

Anstatt nur einen Vakuummotor 1 kann ein Lavage-System 50 auch zwei oder mehr Vakuummotoren 1 aufweisen, deren Vakuumanschlüsse parallel geschaltet sind. Hierdurch wird eine Verstärkung des erzeugten Sprühstrahls bewirkt oder es wird ein höher gepulster Ausstoß erreicht, beispielsweise ein Spülflüssigkeitsstrahl mit doppelter Frequenz erreicht.

Bei allen Ausführungsformen der Vakuummotoren kann auch vorgesehen sein, dass ein zweites Federelement (nicht gezeigt) im oder am Vakuummotor 1 vorgesehen ist, das den Steuerkolben 14 in Richtung des Arbeitskolbens 12 drückt. Bevorzugt ist dazu zwischen der Rückplatte 6 und dem Steuerkolben 14 im hinteren Innenraum eine zweite Druckfeder (nicht gezeigt) angeordnet. Durch diese zweite Druckfeder kann noch sicherer erreicht werden, dass die Ausgangsstellung erreicht wird, wenn kein Vakuum beziehungsweise kein Unterdruck an der Gasauslassöffnung 26 angelegt ist. Zudem wird der Totpunkt (die dritte Position des Steuerkolbens 14) leichter überfahren, bei dem sowohl die Gaseinlassöffnung 24 als auch die Gasauslassöffnung 26 vom Steuerkolben 14 verdeckt und damit geschlossen sind. Ferner kann auch dieses zweite Federelement zum Einstellen der Arbeitsfrequenz des Vakuummotors 1 und damit zur Feinabstimmung des Vakuummotors 1 genutzt werden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Vakuummotor
- 2: Hohlkörper / Wandung
- 4: Deckplatte / Vorderseite des Innenraums
- 6: Rückplatte / Rückseite des Innenraums
- 8: Ausstoßöffnung
- 10: Lippenventil
- 13: Pumpkolben
- 12: Arbeitskolben
- 14: Steuerkolben
- 15: Dichtung / O-Ring
- 16: Abstandhalter / Beabstandungselement
- 17: Durchgang
- 18: Mitnehmerstift
- 19: Belüftungsöffnung
- 20: Mitnehmer
- 22: Federelement / Rückstellelement / Druckfeder / Stahlfeder
- 24: Gaseinlassöffnung
- 26: Gasauslassöffnung
- 28: Flüssigkeitszufuhröffnung
- 30: Abstandhalter
- 34: Rückschlagventil
- 40: Stange / Pleuelstange
- 41: Führungshülse
- 42: Kabel / Schnur
- 43: Strebe
- 50: Lavage-System
- 52: Gehäuse
- 64: Steuerungsventil
- 66: Abzug
- 68: Gasableitung / Verbindung zur Unterdruckquelle
- 70: Spülflüssigkeitszuleitung
- 72: Öffnung
- 74: Griffstück
- 76: Rohr
- 78: Austragöffnung
- 79: Trichter

## Patentansprüche

1. Vakuummotor (1) aufweisend einen Arbeitskolben (12), einen Innenraum, in dem der Arbeitskolben (12) linear beweglich angeordnet ist, ein Rückstellelement (22), das zumindest zeitweise eine in Richtung einer Vorderseite (4) des Innenraums wirkende Kraft auf den Arbeitskolben (12) ausübt, eine Gaseinlassöffnung (24) zum Einspeisen von Umgebungsluft oder eines unter Druck stehenden Gases in den Innenraum und eine Gasauslassöffnung (26) zum Abführen des Gases aus dem Innenraum, wobei die Gasauslassöffnung (26) an eine Unterdruckquelle anschließbar ist, wobei zwischen dem Arbeitskolben (12) und einer Rückseite (6) des Innenraums ein Steuerkolben (14) linear beweglich im Innenraum angeordnet ist, wobei der Steuerkolben (14) in einer ersten Position die Gasauslassöffnung (26) nicht abdeckt und die Gaseinlassöffnung (24) abdeckt und in einer zweiten Position die Gaseinlassöffnung (24) nicht abdeckt und die Gasauslassöffnung (26) abdeckt, der Steuerkolben (14) gegen den Arbeitskolben (12) beweglich gelagert ist und an dem Arbeitskolben (12) und/oder dem Steuerkolben (14) ein Mitnehmerelement (18, 20, 42) und/oder ein Abstandhalter (30) angeordnet ist, wobei das Mitnehmerelement (18, 20, 42) bei einer Bewegung des Arbeitskolbens (12) zur Vorderseite (4) des Innenraums hin den Steuerkolben (14) in die erste Position überführt und wobei das Mitnehmerelement (18, 20, 42) oder der Abstandhalter (30) bei einer Bewegung des Arbeitskolbens (12) zur Rückseite (6) des Innenraums hin den Steuerkolben (14) in die zweite Position überführt, wobei der Steuerkolben (14) in einer dritten Position zwischen der ersten Position und der zweiten Position sowohl die Gaseinlassöffnung (24) als auch die Gasauslassöffnung (26) abdeckt.

2. Vakuummotor (1) nach Anspruch 1, **dadurch gekennzeichnet, dass**
das Mitnehmerelement (18, 20, 42) bei einer Bewegung des Arbeitskolbens (12) vom Steuerkolben (14) weg den Steuerkolben (14) in Richtung des Arbeitskolbens (12) nachzieht und den Steuerkolben (14) in die erste Position überführt und das Mitnehmerelement (18, 20, 42) oder der Abstandhalter (30) bei einer Bewegung des Arbeitskolbens (12) zum Steuerkolben (14) hin den Steuerkolben (14) in Richtung der Rückseite (6) drückt und den Steuerkolben (14) in die zweite Position überführt.

3. Vakuummotor (1) nach einem der Ansprüche 1 oder 2, **dadurch gekennzeichnet, dass**
das Mitnehmerelement (18,20,42) bei einer Bewegung des Arbeitskolbens (12) vom Steuerkolben (14) weg den Steuerkolben (14) in Richtung des Arbeitskolbens (12) nachzieht und den Steuerkolben (14) in die erste Position überführt und das Mitnehmerelement (18, 20, 42) oder der Abstandhalter (30) bei einer Bewegung des Arbeitskolbens (12) zum Steuerkolben (14) hin den Steuerkolben (14) in Richtung der Rückseite (6) drückt und den Steuerkolben (14) in die zweite Position überführt.

4. Vakuummotor (1) nach einem der vorangehenden Ansprüche, **dadurch gekennzeichnet, dass**
das Rückstellelement (22) eine elastische Druckfeder (22) ist, die im Innenraum zwischen dem Arbeitskolben (12) und der Rückseite (6) des Innenraums angeordnet ist.

5. Vakuummotor (1) nach einem der vorangehenden Ansprüche, **dadurch gekennzeichnet, dass**
der Steuerkolben (14) in der ersten Position von dem Rückstellelement (22) über das Mitnehmerelement (18, 20, 42) in Richtung der Vorderseite (4) gezogen ist, wobei die Gasauslassöffnung (26) in den Zwischenraum zwischen dem Steuerkolben (14) und der Rückseite (6) des Innenraums mündet.

6. Vakuummotor (1) nach einem der vorangehenden Ansprüche, **dadurch gekennzeichnet, dass**
in dem Steuerkolben (14) wenigstens ein gasdurchlässiger Durchgang (17) angeordnet ist, die die dem Arbeitskolben (12) zugewandte Vorderseite des Steuerkolbens (14) mit der der Rückseite (6) des Innenraums zugewandten Rückseite des Steuerkolbens (14) gasdurchlässig verbindet.

7. Vakuummotor (1) nach einem der vorangehenden Ansprüche, **dadurch gekennzeichnet, dass**
in der der Rückseite (6) gegenüberliegenden Vorderseite (4) des Innenraums eine Ausstoßöffnung (8) vorgesehen ist und in der Vorderseite (4) und/oder in der seitlichen Wandung (2) des Innenraums eine Flüssigkeitszufuhröffnung (28) angeordnet ist, die zumindest zeitweise nicht vom Arbeitskolben (12) abgedeckt ist und die im nicht abgedeckten Zustand zwischen dem Arbeitskolben (12) und der Vorderseite (4) des Innenraums angeordnet ist.

8. Vakuummotor (1) nach Anspruch 7, **dadurch gekennzeichnet, dass**
die Ausstoßöffnung (8) durch ein Ventilelement (10), insbesondere ein Lippenventil (10), mit der Umgebung verbunden ist, wobei das Ventilelement (10) bei einem ausreichenden Überdruck gegenüber dem Umgebungsdruck geöffnet und ansonsten geschlossen ist, und dass an der Flüssigkeitszufuhröffnung (28) ein Rohr oder eine Schlauch mit einem Rückschlagventil (34) angeschlossen ist, das sich bei einem Unterdruck im Innenraum zwischen dem Arbeitskolben (12) und der Vorderseite (4) des Innenraums öffnet und eine Flüssigkeitszufuhr in den Innenraum ermöglicht.

9. Vakuummotor (1) nach einem der vorangehenden Ansprüche, **dadurch gekennzeichnet, dass**
das Mitnehmerelement (18, 20, 42) eine Schnur (42), ein Kabel (42), ein Faden, eine Kette oder eine elastische Feder ist, die am Arbeitskolben (12) und am Steuerkolben (14) befestigt ist oder das Mitnehmerelement (18, 20, 42) eine Stange (18), eine Schnur, ein Kabel, ein Faden, eine Kette oder eine elastische Feder aufweist, die am Arbeitskolben (12) oder am Steuerkolben (14) befestigt ist und an der ein Mitnehmer (20) befestigt ist, der bei der periodischen Bewegung des Arbeitskolbens (12) in einen Vorsprung im Arbeitskolben (12) oder im Steuerkolben (14) greift, wobei bevorzugt das Mitnehmerelement (18, 20, 42) durch eine am Arbeitskolben (12) befestigte Stange (18) gebildet wird, die durch eine Durchführung im Steuerkolben (14) reicht und an der der Mitnehmer (20) befestigt ist, der nicht durch die Durchführung im Steuerkolben (14) passt und der auf der Rückseite der Durchführung des Steuerkolbens (14) in diese eingreift um den Steuerkolben (14) mitzuziehen, wenn der Arbeitskolben (12) dazu ausreichend weit von dem Steuerkolben (14) entfernt ist und sich in Richtung von diesem weg bewegt.

10. Vakuummotor (1) nach einem der vorangehenden Ansprüche, **dadurch gekennzeichnet, dass**
der Arbeitskolben (12) zwei unterschiedlich große Querschnittsflächen senkrecht zur linearen Bewegungsrichtung des Arbeitskolbens (12) aufweist, wobei der Innenraum dazu passende Innenwände mit unterschiedlichen Querschnittsflächen aufweist und die Querschnittsfläche auf der der Rückseite (6) zugewandten Seite des Arbeitskolbens (12) zumindest 100% größer ist als die Querschnittsfläche der gegenüberliegenden Vorderseite des Arbeitskolbens (12), bevorzugt die Querschnittsfläche auf der, der Rückseite (6) zugewandten Seite des Arbeitskolbens (12) zumindest vier Mal so groß ist wie die Querschnittsfläche der gegenüberliegenden Vorderseite des Arbeitskolbens (12).

11. Lavage-System (50) aufweisend zumindest einen Vakuummotor (1) nach einem der vorangehenden Ansprüche, bei dem mit dem Vakuummotor (1) oder den Vakuummotoren (1) ein periodischer Sprühstoß mit einer Flüssigkeit erzeugbar ist.

12. Verwendung eines Vakuummotors (1) nach einem der Ansprüche 1 bis 10 als Motor für einen Klopfermotor, einen Vibrationsmotor, als Antrieb für eine Dosiervorrichtung, als Rüttelmotor oder als Pumpe, insbesondere als Schmiermittelpumpe.

13. Verfahren zum Erzeugen einer periodischen Bewegung mit einem Vakuum oder einem Unterdruck unter Verwendung eines Vakuummotors, insbesondere unter Verwendung eines Vakuummotors (1) nach einem der Ansprüche 1 bis 10, **dadurch gekennzeichnet, dass** in einem Ausgangszustand ein Arbeitskolben (12) und ein Steuerkolben (14) in einem Innenraum des Vakuummotors linear beweglich angeordnet werden und voneinander mit einem ersten Abstand beabstandet werden, wobei der Steuerkolben (14) eine Gaseinlassöffnung (24) verschließt und eine Gasauslassöffnung (26) offen liegt; dass Gas zwischen dem Arbeitskolben (12) und dem Steuerkolben (14) und zwischen dem Arbeitskolben (12) und einer Rückseite (6) des Innenraums durch die Gasauslassöffnung (26) aus dem Innenraum herausgezogen wird;
der Arbeitskolben (12) durch die Druckdifferenz zwischen dem auf der Vorderseite und der Rückseite des Arbeitskolbens (12) lastenden Gasdruck in Richtung der Rückseite (6) des Innenraums zum Steuerkolben (14) hin beschleunigt und bewegt wird, wobei sich der Abstand zwischen dem Arbeitskolben (12) und dem Steuerkolben (14) verringert und ein Rückstellelement (22), das zumindest zeitweise eine in Richtung der Vorderseite des Innenraums wirkende Kraft auf den Arbeitskolben ausübt, durch die Bewegung des Arbeitskolbens (12) Energie aufnimmt und speichert; der Steuerkolben (14) über ein an dem Arbeitskolben und/oder dem Steuerkolben angeordnetes Mitnehmerelement (18, 20, 42) oder einen an dem Arbeitskolben und/oder dem Steuerkolben angeordneten Abstandhalter (30) von dem Arbeitskolben (12) mit geschoben wird, bevorzugt ab Erreichen eines zweiten Abstands;
der Steuerkolben (14) die Gasauslassöffnung (26) aufgrund der Bewegung des Steuerkolbens (14) verschließt;
der Steuerkolben (14) die Gaseinlassöffnung (24) aufgrund der Bewegung des Steuerkolbens (14) öffnet; dass ein Druckgas oder die umgebende Luft durch die Gaseinlassöffnung (24) in den Innenraum strömt;
der Arbeitskolben (12) durch das Rückstellelement (22) in Richtung der Vorderseite (4) des Innenraums beschleunigt wird;
der Steuerkolben (14) über das Mitnehmerelement (18, 20, 42) von dem Arbeitskolben (12) mitgezogen wird und diesen in Richtung der Vorderseite (4) des Innenraums bewegt, bevorzugt ab Erreichen eines ersten Abstands;
durch die umgekehrte Bewegung des Steuerkolbens (14) die Gaseinlassöffnung (24) wieder geschlossen wird und
durch die umgekehrte Bewegung des Steuerkolbens (14) die Gasauslassöffnung (26) wieder geöffnet wird, so dass erneut das Gas zwischen dem Arbeitskolben (12) und der Rückseite (6) des Innenraums aus dem Innenraum heraus gezogen wird.

14. Verfahren nach Anspruch 13, **dadurch gekennzeichnet, dass**
der zweite Abstand zwischen dem Arbeitskolben (12) und dem Steuerkolben (14) mit Hilfe des Abstandhalters (30) oder des Mitnehmerelements (18, 20, 42) eingestellt wird und der erste Abstand zwischen dem Arbeitskolben (12) und dem Steuerkolben (14) mit dem Mitnehmerelement (18, 20, 42) eingestellt wird, vorzugsweise durch die Länge des Mitnehmerelements (18, 20, 42).

15. Verfahren zum Erzeugen eines Sprühstoßes aufweisend die Verfahrensschritte nach einem der Ansprüche 13 oder 14, **dadurch gekennzeichnet, dass**
bei einer Bewegung des Arbeitskolbens (12), insbesondere eines Pumpkolbens (13) des Arbeitskolbens (12), weg von der Rückseite (6) des Innenraums eine Spülflüssigkeit oder ein Flüssigkeit-Gas-Gemisch aus dem Raum zwischen dem Arbeitskolben (12) und der Vorderseite (4) des Innenraums durch eine Ausstoßöffnung an der Vorderseite (4) des Innenraums hinaus gepresst wird und
bei einer Bewegung des Arbeitskolbens (12), insbesondere des Pumpkolbens (13) des Arbeitskolbens (12), auf die Rückseite (6) des Innenraums zu eine Flüssigkeit oder ein Flüssigkeit-Gas-Gemisch in den Raum zwischen dem Arbeitskolben (12), insbesondere dem Pumpkolben (13), und der Vorderseite (4) des Innenraums durch eine Flüssigkeitszufuhröffnung (28) hinein gedrückt oder gezogen wird.

16. Verfahren nach Anspruch 15, **dadurch gekennzeichnet, dass**
bei der Bewegung des Arbeitskolbens (12) auf die Vorderseite (4) des Innenraums zu durch den Druck in dem Raum zwischen dem Arbeitskolben (12) und der Vorderseite (4) des Innenraums ein Ventil (10) an der Ausstoßöffnung (8) geöffnet wird und/oder offen gehalten wird und ein an die Flüssigkeitszufuhröffnung (28) angeschlossenes Rückschlagventil (34) geschlossen wird und/oder geschlossen gehalten wird und
bei der Bewegung des Arbeitskolbens (12) auf die Rückseite (6) des Innenraums zu durch den geringeren Druck in dem Raum zwischen dem Arbeitskolben (12) und der Vorderseite (4) des Innenraums das Ventil (10) an der Ausstoßöffnung (8) geschlossen wird und/oder geschlossen gehalten wird und das an die Flüssigkeitszufuhröffnung (28) angeschlossene Rückschlagventil (34) geöffnet wird und/oder offen gehalten wird.

## Claims

1. Vacuum motor (1) comprising a working plunger (12), an internal space, in which
the working plunger (12) is arranged such that it is mobile in linear direction, a resetting element (22) that exerts, at least for part of the time, a force on the working plunger (12) that acts in the direction of a front side (4) of the internal space, a gas inlet opening (24) for supplying ambient air or a compressed gas into the internal space, and a gas outlet opening (26) for discharging the gas from the internal space, whereby the gas outlet opening (26) is connectable to a negative pressure source, whereby a control plunger (14) is arranged between the working plunger (12) and a rear side (6) of the internal space such as to be mobile in linear direction in the internal space, whereby the control plunger (14), in a first position, does not cover the gas outlet opening (26) and covers the gas inlet opening (24) and, in a second position, does not cover the gas inlet opening (24) and covers the gas outlet opening (26), the control plunger (14) is supported as in a bearing such as to be mobile with respect to the working plunger (12), and a catch element (18, 20, 42) and/or a spacer (30) is arranged on said working plunger (12) and/or control plunger (14), whereby the catch element (18, 20, 42), upon a motion of the working plunger (12) towards the front side (4) of the internal space, transfers the control plunger (14) into the first position, and whereby the catch element (18, 20, 42) or the spacer (30), upon a motion of the working plunger (12) towards the rear side (6) of the internal space, transfers the control plunger (14) into the second position, wherein the control plunger (14), in a third position between the first position and the second position, covers both the gas inlet opening (24) and the gas outlet opening (26).

2. Vacuum motor (1) according to claim 1, **characterised in that**
the catch element (18, 20, 42), upon a motion of the working plunger (12) away from the control plunger (14), pulls the control plunger (14) along in the direction of the working plunger (12) and transfers the control plunger (14) into the first position, and the catch element (18, 20, 42) and/or the spacer (30), upon a motion of the working plunger (12) towards the control plunger (14), pushes the control plunger (14) in the direction of the rear side (6) and transfers the control plunger (14) into the second position.

3. Vacuum motor (1) according to claim 1 or 2, **characterised in that**
the catch element (18, 20, 42), upon a motion of the working plunger (12) away from the control plunger (14), pulls the control plunger (14) along in the direction of the working plunger (12) and transfers the control plunger (14) into the first position, and the catch element (18, 20, 42) and/or the spacer (30), upon a motion of the working plunger (12) towards the control plunger (14), pushes the control plunger (14) in the direction of the rear side (6) and transfers the control plunger (14) into the second position.

4. Vacuum motor (1) according to any one of the preceding claims, **characterised in that**
the resetting element (22) is an elastic compression spring (22) that is arranged in
the internal space between the working plunger (12) and the rear side (6) of the internal space.

5. Vacuum motor (1) according to any one of the preceding claims, **characterised in that**
the control plunger (14), in the first position, is pulled via the catch element (18, 20, 42) in the direction of the front side (4) by the resetting element (22), whereby
the gas outlet opening (26) opens into the intervening space between the control plunger (14) and the rear side (6) of the internal space.

6. Vacuum motor (1) according to any one of the preceding claims, **characterised in that**
at least one gas-permeable passage (17) is arranged in the control plunger (14) and connects the front side of the control plunger (14) facing the working plunger (12) to the rear side of the control plunger (14) facing the rear side (6) of the internal space in gas-impermeable manner.

7. Vacuum motor (1) according to any one of the preceding claims, **characterised in that**
an ejection opening (8) is provided in the front side (4) of the internal space opposite from the rear side (6), and a liquid supply opening (28) is arranged in the front side (4) and/or in the lateral wall (2) of the internal space and the opening is not covered by the working plunger (12) at least for part of the time and, in the non-covered state, is arranged between the working plunger (12) and the front side (4) of the internal space.

8. Vacuum motor (1) according to claim 7, **characterised in that**
the ejection opening (8) is connected to the surroundings by a valve element (10), in particular a lip valve (10), whereby the valve element (10) is opened in the presence of sufficient over-pressure as compared to the ambient pressure and is closed otherwise, and **in that** a tube or a hose with a non-return valve (34) is connected on the liquid supply opening (28) and opens in the presence of a negative pressure in the internal space between the working plunger (12) and the front side (4) of the internal space and thus enables liquid to be supplied into the internal space.

9. Vacuum motor (1) according to any one of the preceding claims, **characterised in that**
the catch element (18, 20, 42) is a string (42), a cable (42), a thread, a chain or an elastic spring that is attached to the working plunger (12) and to the control plunger (14) or the catch element (18, 20, 42) comprises a rod (18), a string, a cable, a thread, a chain or an elastic spring that is attached to the working plunger (12) or to the control plunger (14) and has a catch (20) attached to it that engages, upon the periodical motion of the working plunger (12), a projection in the working plunger (12) or in the control plunger (14), whereby the catch element (18, 20, 42) preferably is provided by a rod (18) that is attached to the working plunger (12) and extends through a feed-through in the control plunger (14) and has a catch (20) attached to it that does not fit through the feed-through in the control plunger (14) and engages the feed-through on the rear side of the feed-through of the control plunger (14) in order to pull the control plunger (14) along, when the working plunger (12) is sufficiently far away from the control plunger (14) for this purpose and moves in the direction away from the control plunger (14).

10. Vacuum motor (1) according to any one of the preceding claims, **characterised in that**
the working plunger (12) comprises two differently-sized cross-sectional surfaces perpendicular to the linear motion direction of the working plunger (12), whereby the internal space comprises matching internal walls with different cross-sectional surfaces and the cross-sectional surface on the side of the working plunger (12) facing the rear side (6) is at least 100% larger than the cross-sectional surface of the opposite front side of the working plunger (12), preferably the cross-sectional surface on the side of the working plunger (12) facing the rear side (6) is at least four times the size of the cross-sectional surface of the opposite front side of the working plunger (12).

11. Lavage system (50) comprising at least one vacuum motor (1) according to any one of the preceding claims, in which the vacuum motor (1) or the vacuum motors (1) can be used to generate a periodical spray puff of a liquid.

12. Use of a vacuum motor (1) according to any one of the claims 1 to 15 as motor for a lavage system (50), a rapping motor, a vibration motor, as drive for a dosing facility, as shaker motor or as pump, in particular as lubricant pump.

13. Method for generating a periodical motion by means of a vacuum or negative pressure using a vacuum motor, in particular involving the use of a vacuum motor (1) according to anyone of the claims 1 to 10, **characterised in that**
the working plunger (12) and the control plunger (14) are arranged linear movable in an internal space of the vacuum motor, and, in a starting state, are situated in the internal space such as to be at a first distance from each other, whereby the control plunger (14) closes the gas inlet opening (24) and the gas outlet opening (26) is open;
the gas between the working plunger (12) and the control plunger (14) and between the working plunger (12) and the rear side (6) of the internal space is drawn out of the internal space through the gas outlet opening (26);
the working plunger (12) is accelerated and moved in the direction of the rear side (6) of the internal space towards the control plunger (14) by the pressure difference between the gas pressure exerting on the front side versus the gas pressure exerting on the rear side of the working plunger (12), whereby the distance between the working plunger (12) and the control plunger (14) decreases and a resetting element (22) that exerts, at least for part of the time, a force on the working plunger (12) that acts in the direction of a front side (4) of the internal space takes up and stores energy due to the motion of the working plunger (12); the control plunger (14) is pushed along by the working plunger (12) by means of the catch element (18, 20, 42) arranged on the working plunger or the control plunger or a spacer (30) arranged on the working plunger or the control plunger, preferably as soon as a second distance is reached;
the control plunger (14) closes the gas outlet opening (26) due to the motion of the control plunger (14);
the control plunger (14) opens the gas inlet opening (24) due to the motion of the control plunger (14);
a compressed gas or ambient air flows through the gas inlet opening (24) into the internal space;
the resetting element (22) accelerates the working plunger (12) in the direction of the front side (4) of the internal space;
the control plunger (14) is pulled along by the working plunger (12) by means of the catch element (18, 20, 42) and moves the same in the direction of the front side (4) of the internal space, preferably as soon as a first distance is reached; the gas inlet opening (24) is closed again by the reverse motion of the control plunger (14); and
the reverse motion of the control plunger (14) opens the gas outlet opening (26) again such that the gas between the working plunger (12) and the rear side (6) of the internal space is drawn out of the internal space again.

14. Method according to claim 15, **characterised in that**
the second distance between working plunger (12) and control plunger (14) is adjusted by means of the spacer (30) or the catch element (18, 20, 42) and the first distance between working plunger (12) and control plunger (14) is adjusted by the catch element (18, 20, 42), preferably by means of the length of the catch element (18, 20, 42).

15. Method for generating a spray puff comprising the procedural steps according to any one of the claims 13 or 14, **characterised in that**,
upon a motion of the working plunger (12), in particular of a pump plunger (13) of the working plunger (12), away from the rear side (6) of the internal space, a rinsing liquid or a liquid-gas mixture is ejected from the space between the working plunger (12) and the front side (4) of the internal space through an ejection opening on the front side (4) of the internal space, and,
upon a motion of the working plunger (12), in particular of the pump plunger (13) of the working plunger (12), towards the rear side (6) of the internal space, a liquid or a liquid-gas mixture is pushed or pulled through a liquid supply opening (28) into the space between the working plunger (12), in particular of a pump plunger (13), and the front side (4) of the internal space.

16. Method according to claim 15, **characterised in that**
upon the motion of the working plunger (12) towards the front side (4) of the internal space, the pressure in the space between the working plunger (12) and the front side (4) of the internal space opens and/or keeps open a valve (10) at the ejection opening (8) and closes and/or keeps closed a non-return valve (34) connected to the liquid supply opening (28), and, upon the motion of the working plunger (12) towards the rear side (6) of the internal space, the lesser pressure in the space between the working plunger (12) and the front side (4) of the internal space closes and/or keeps closed the valve (10) on the ejection opening (8) and opens and/or keeps open the non-return valve (34) connected to the liquid supply opening (28).

## Revendications

1. Moteur à vide (1) présentant un piston de travail (12), une chambre intérieure, dans laquelle le piston de travail (12) est disposé en étant mobile linéairement, un élément de rappel (22) qui exerce au moins temporairement une force sur le piston de travail (12) agissant en direction d'une paroi avant (4) de la chambre intérieure, un orifice d'entrée de gaz (24) pour l'approvisionnement en air environnant ou en un gaz se trouvant sous pression dans la chambre intérieure et un orifice de sortie de gaz (26) pour l'évacuation du gaz à partir de la chambre intérieure, où l'orifice de sortie de gaz (26) peut être raccordé à une source de pression négative, où, entre le piston de travail (12) et la paroi arrière (6) de la chambre intérieure, un piston de commande (14) est disposé en étant mobile linéairement dans la chambre intérieure, où le piston de commande (14) ne recouvre pas l'orifice de sortie de gaz (26) dans une première position et recouvre l'orifice d'entrée de gaz (24) et, dans une deuxième position, ne recouvre pas l'orifice d'entrée de gaz (24) et recouvre l'orifice de sortie de gaz (26), le piston de commande (14) est logé en étant mobile contre le piston de travail (12) et un élément d'entraînement (18, 20, 42) et/ou une entretoise (30) est disposée sur le piston de travail (12) et/ou le piston de commande(14), où l'élément d'entraînement (18, 20, 42), lors d'un mouvement du piston de travail (12) vers la paroi avant (4) de la chambre intérieure, transfère le pison de commande (14) dans la première position et où l'élément d'entraînement (18, 20, 42), ou l'entretoise (30), transfère le piston de commande (14) dans la deuxième position lors d'un mouvement du piston de travail (12) vers la paroi arrière (6) de la chambre intérieure, où le piston de commande (14) recouvre à la fois l'orifice d'entrée de gaz (24) et l'orifice de sortie de gaz (26) dans une troisième position entre la première position et la deuxième position.

2. Moteur à vide (1) selon la revendication 1, **caractérisé en ce que**
l'élément d'entraînement (18, 20, 42), lors d'un mouvement du piston de travail (12) s'éloignant du piston de commande (14), entraîne le piston de commande (14) en direction du piston de travail (12) et transfère le piston de commande (14) dans la première position, et l'élément d'entraînement (18 20, 42), ou l'entretoise (30), lors d'un mouvement du piston de travail (12) vers le piston de commande (14), presse le piston de commande (14) en direction de la paroi arrière (6) et transfère le piston de commande (14) dans la deuxième position.

3. Moteur à vide (1) selon la revendication 1 ou 2, **caractérisé en ce que**
l'élément d'entraînement (18, 20, 42), lors d'un mouvement du piston de travail (12) l'éloignant du piston de commande (14), entraîne le piston de commande (14) en direction du piston de travail (12) et transfère le piston de commande (14) dans la première position, et l'élément d'entraînement (18, 20, 42), ou l'entretoise (30), lors d'un mouvement du piston de travail (12) en direction du piston de commande (14), presse le piston de commande (14) en direction de la paroi arrière (6) et transfère le piston de commande (14) dans la deuxième position.

4. Moteur à vide (1) selon l'une des revendications précédentes, **caractérisé en ce que**
l'élément de rappel (22) est un ressort de compression (22) élastique qui est disposé dans la chambre intérieure entre le piston de travail (12) et la paroi arrière (6) de la chambre intérieure.

5. Moteur à vide (1) selon l'une des revendications précédentes, **caractérisé en ce que**
le piston de commande (14) dans la première position est tiré à partir de
l'élément de rappel (22) en direction de la paroi avant (4) par le biais de
l'élément d'entraînement (18, 20, 42), où l'orifice de sortie de gaz (26) débouche dans la chambre intermédiaire entre le piston de commande (14) et la paroi arrière (6) de la chambre intérieure.

6. Moteur à vide (1) selon l'une des revendications précédentes, **caractérisé en ce que**
dans le piston de commande (14), il y a au moins un passage (17) perméable aux gaz, qui relie de manière perméable aux gaz la paroi avant du piston de commande (14) orientée vers le piston de travail (12) avec la paroi arrière du piston de commande (14) orientée vers la paroi arrière (6) de la chambre intérieure.

7. Moteur à vide (1) selon l'une des revendications précédentes, **caractérisé en ce que**
dans la paroi avant (4) de la chambre intérieure située à l'opposé de la paroi arrière (6), un orifice d'expulsion (8) est prévu et dans la paroi avant (4) et/ou dans la paroi latérale (2) de la chambre interne, il y a un orifice d'alimentation en liquide (28), qui au moins temporairement n'est pas recouvert par le piston de travail (12) et qui est disposé à l'état non recouvert entre le piston de travail (12) et la paroi avant (4) de la chambre intérieure.

8. Moteur à vide (1) selon la revendication 7, **caractérisé en ce que** l'orifice d'expulsion (8) est relié à l'environnement par un élément de soupape (10), notamment un clapet à lèvres (10), où l'élément de soupape (10) est ouvert pour une surpression suffisante par rapport à la pression environnante et est par ailleurs fermé, et qu'au niveau de l'orifice d'alimentation en liquide (28), un tube ou un tuyau est branché avec un clapet anti-retour (34), qui s'ouvre pour une pression négative dans la chambre intérieure entre le piston de travail (12) et la paroi avant (4) de la chambre intérieure et permet une alimentation de liquide dans la chambre intérieure.

9. Moteur à vide (1) selon l'une des revendications précédentes, **caractérisé en ce que**
l'élément d'entraînement (18, 20, 42) est une ficelle (42), un câble (42), un fil, une chaîne ou un ressort élastique qui est fixé sur le piston de travail (12) et sur le piston de commande (14), ou l'élément d'entraînement (18, 20, 42) présente une tige (18), une ficelle, un câble, une chaîne ou un ressort élastique qui est fixé sur le piston de travail (12) ou sur le piston de commande (14), et auquel un système d'entraînement (20) est fixé qui se met en prise dans une saillie dans le piston de travail (12) ou dans le piston de commande (14) lors du mouvement périodique du piston de travail (12), où, de préférence, l'élément d'entraînement (18, 20, 42) est formé par une tige (18) fixée sur le piston de travail (12), qui va jusqu'au piston de commande (14) par un passage et sur laquelle le système d'entrainement (20) est fixé, qui ne peut pas passer par le passage dans le piston de commande (14) et laquelle se met en prise sur la paroi arrière du passage du piston de commande afin d'entraîner le piston de commande (14) lorsque le piston de travail (12) est suffisamment éloigné du piston de commande (14) et se déplace en s'en éloignant.

10. Moteur à vide (1) selon l'une des revendications précédentes, **caractérisé en ce que**
le piston de travail (12) présente deux surfaces de sections transversales de différentes tailles perpendiculairement par rapport à la direction du mouvement du piston de travail (12), où la chambre intérieure présente de manière complémentaire des parois intérieures correspondantes avec des surfaces de sections transversales diverses et la surface de section transversale sur le côté du piston de travail (12) orienté vers la paroi arrière (6) est supérieure d'au moins 100 % à la surface de la section transversale du côté avant du piston de travail (12) située en face, de préférence, la surface de la section transversale sur laquelle le côté du piston de travail (12) orienté vers la paroi arrière (6) est au moins quatre fois aussi grande que la surface de la section transversale du côté avant du piston de travail (12) située en face.

11. Système de lavage (50) présentant au moins un moteur à vide (1) selon l'une des revendications précédentes, dans lequel, avec le moteur à vide (1) ou les moteurs à vide, une poussée de pulvérisation périodique avec un liquide est réalisable.

12. Utilisation d'un moteur à vide (1) selon l'une des revendications 1 à 10 en tant que moteur pour un moteur de batteur, un moteur de vibration, en tant qu'entraînement pour un dispositif de dosage, en tant que moteur vibrant ou en tant que pompe, notamment en tant que pompe à lubrifiant.

13. Procédé de génération d'un mouvement périodique avec un vide ou une pression négative moyennant l'emploi d'un moteur à vide, notamment, moyennant l'emploi d'un moteur à vide (1) selon l'une des revendications 1 à 10, **caractérisé en ce que**
dans un état de départ, un piston de travail (12) et un piston de commande (14) dans une chambre intérieure du moteur à vide sont disposés mobiles linéairement et sont espacés l'un de l'autre à une première distance, où le piston de commande (14) ferme un orifice d'entrée de gaz (24) et un orifice de sortie de gaz (26) est ouvert ; que du gaz peut être aspiré à partir de la chambre intérieure entre le piston de travail (12) et le piston de commande (14) et entre le piston de travail (12) et une paroi arrière (6) de la chambre intérieure ;
le piston de travail (12) est accéléré et déplacé en direction de la paroi arrière (6) de la chambre intérieure vers le piston de commande du fait de la différence de pression entre la pression de gaz agissant sur le côté avant et le côté arrière du piston de travail (12), où la distance entre le piston de travail (12) et le piston de commande (14) diminue et un élément de rappel (22) qui exerce une force agissant au moins temporairement sur le piston de travail en direction de la paroi avant de la chambre intérieure, emmagasine et accumule de l'énergie par le mouvement du piston de travail (12) ;
le piston de commande (14) est entraîné par le biais d'un élément d'entraînement (18, 20, 42) disposé sur le piston de travail et/ou sur le piston de commande, ou une entretoise (30) disposée sur le piston de travail et/ou le piston de commande est entraînée par le piston de travail (12), de préférence, après avoir atteint un deuxième état ;
le piston de commande (14) ferme l'orifice de sortie de gaz (26) en raison du mouvement du piston de commande (14) ;
le piston de commande (14) ouvre l'orifice d'entrée de gaz en raison du mouvement du piston de commande (14) ;
qu'un gaz sous pression ou l'air environnant entre dans la chambre intérieure par l'orifice d'entrée de gaz (24) ;
le piston de travail (12) est accéléré en direction de la paroi avant (4) de la chambre intérieure par l'élément de rappel (22) ;
le piston de commande (14) est entraîné par le biais de l'élément d'entraînement (18, 20, 42) à partir du piston de travail (12) et déplace celui-ci en direction de la paroi avant (4) de la chambre intérieure, de préférence, après avoir atteint la première distance ;
l'orifice d'entrée de gaz (24) est de nouveau fermé par le mouvement inverse du piston de commande (14) et
l'orifice de sortie de gaz (26) est de nouveau ouvert par le mouvement inverse du piston de commande (14) de sorte que de nouveau, le gaz entre le piston de travail (12) et la paroi arrière (6) de la chambre intérieure est aspiré hors de la chambre intérieure.

14. Procédé selon la revendication 13, **caractérisé en ce que**
la deuxième distance entre le piston de travail (12) et le piston de commande (14) est réglée à l'aide de l'entretoise (30) ou de l'élément d'entraînement (18, 20, 42) et la première distance entre le piston de travail (12) et le piston de commande (14) est réglée avec l'élément d'entraînement (18, 20, 42), de préférence par la longueur de l'élément d'entraînement (18, 20, 42).

15. Procédé de réalisation d'un jet de pulvérisation présentant les étapes de procédé selon l'une des revendications 13 ou 14, **caractérisé en ce que** lors d'un mouvement du piston de travail (12), notamment d'un piston de pompe (13) du piston de travail (12), s'éloignant de la paroi arrière (6) de la chambre intérieure, un liquide de lavage ou un mélange de liquide et de gaz est pressé hors de la chambre entre le piston de travail (12) et la paroi avant (4) de la chambre intérieure par un orifice d'expulsion sur la paroi avant (4) de la chambre intérieure et
lors d'un mouvement du piston de travail (12), notamment d'un piston de pompe (13) du piston de travail (12), vers la paroi arrière (6) de la chambre intérieure, un liquide ou un mélange de liquide et de gaz est pressé ou aspiré à l'intérieur dans la chambre entre le piston de travail (12), notamment le piston de pompe (13), et la paroi avant (4) de la chambre intérieure par un orifice d'alimentation de liquide (28).

16. Procédé selon la revendication 15, **caractérisé en ce que**
lors du mouvement du piston de travail (12) vers la paroi avant (4) de la chambre intérieure, une soupape (10) au niveau de l'orifice d'expulsion (8) est ouverte et/ou est maintenue ouverte par la pression dans la chambre entre le piston de travail (12) et la paroi avant (4) de la chambre intérieure, et un clapet anti-retour (34) raccordé au niveau de l'orifice d'alimentation en liquide (28) est fermé et/ou est maintenu fermé, et
lors du mouvement du piston de travail (12) en direction de la paroi arrière (6) de la chambre intérieure, la chambre entre le piston de travail (12) et la paroi avant (4) de la chambre intérieure, la soupape (10) au niveau de l'orifice d'expulsion (8) est fermée et/ou est maintenue fermée par la pression moindre dans la chambre entre le piston de travail (12) et la paroi avant (4) de la chambre intérieure et le clapet anti-retour (34) raccordé sur l'orifice d'alimentation en liquide (28) est ouvert et/ou est maintenu ouvert.
